(19) **Europäisches Patentamt**
**European Patent Office**
**Office européen des brevets**

(11) Veröffentlichungsnummer: **0 162 812 B1**

(12) # EUROPÄISCHE PATENTSCHRIFT

(45) Veröffentlichungstag der Patentschrift: **01.09.93**

(21) Anmeldenummer: **85810232.0**

(22) Anmeldetag: **15.05.85**

(51) Int. Cl.5: **C07K 15/12**, C07K 3/18, C12P 21/02, C07K 3/28, C12P 21/00, C12N 15/00, C12N 5/00, G01N 33/577, G01N 33/68, A61K 37/02, A61K 39/395, //(C12P21/02, C12R1:91),(C12P21/00, C12R1:91)

(54) **Lymphokin in reiner Form, neue monoklonale Antikörper, Hydridoma-Zellinien, Verfahren und Anwendungen.**

(30) Priorität: 24.05.84 CH 2557/84
07.08.84 CH 3786/84
14.11.84 CH 5446/84
05.02.85 CH 512/85

(43) Veröffentlichungstag der Anmeldung:
**27.11.85 Patentblatt 85/48**

(45) Bekanntmachung des Hinweises auf die Patenterteilung:
**01.09.93 Patentblatt 93/35**

(84) Benannte Vertragsstaaten:
**AT BE CH DE FR GB IT LI LU NL SE**

(56) Entgegenhaltungen:
EP-A- 0 020 090
DD-A- 230 876
GB-A- 2 009 927

(73) Patentinhaber: **CIBA-GEIGY AG**
**Klybeckstrasse 141**
**CH-4002 Basel(CH)**

(72) Erfinder: **Sorg, Clemens, Prof. Dr.**
**Alhardstrasse 21**
**D-4400 Münster(DE)**
Erfinder: **Burmeister, Gerd, Dr.**
**Im Schänzli 103 B**
**CH-4132 Muttenz(CH)**
Erfinder: **Tarcsay, Lajos, Dr.**
**Muttenzerstrasse 25**
**D-7889 Grenzach-Wyhlen(DE)**
Erfinder: **Wiesendanger, Walter**
**Steingrubenweg 12**
**CH-4142 Münchenstein(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3. 10/3.6/3.3. 1)

THE JOURNAL OF IMMUNOLOGY, Band 112, Nr. 2, Februar 1974, Seiten 675-682, The Williams & Wilkins Co., US; P.S. PAPAGEORGIOU et al.: "Similarity of migration inhibitory factor(s) produced by human lymphoid cell line and phytohemagglutinin and tuberculin-stimulated human peripheral lymphocytes"

IMMUNOBIOLOGY, Band 160, Nr. 1, 1981, Seite 15, Gustav Fischer Verlag, Stuttgart, DE; G. BURMEISTER et al.: "Chemical characterization of human macrophage migration inhibitory factors"

NATURE, Band 256, 7. August 1975, Seiten 495-497; G. KÖHLER et al.: "Continuous cultures of fused cells secreting antibody of predefined specificity"

ABSTRACTS FROM FOURTH INTERNATIONAL LYMPHOKINE WORKSHOP, 17.-21. Oktober 1984, Band 3, Nr. 4, Seite 236; G. BURMEISTER et al.: "Production and characterization of a monoclonal antibody to human macrophage migration inhibitory factors (MIF)"

## Beschreibung

Die Erfindung betrifft gereinigten Human-Makrophagen-Migrations-inhibitionsfaktor (Human-MIF), der Proteine humanen Ursprungs enthält, die vom monoklonalen Antikörper 1C5 gegen Human-MIF erkannt und gebunden werden, und seine Einzelproteine, ein Verfahren zur Reinigung von Human-MIF und zur Isolierung seiner Einzelproteine, neue monoklonale Antikörper gegen Human-MIF, Derivate davon, Verfahren zur Herstellung dieser Antikörper und ihrer Derivate, Hybridoma-Zellinien, die diese Antikörper produzieren, Verfahren zur Herstellung besagter Hybridoma-Zellinien, die Verwendung der monoklonalen Antikörper und ihrer Derivate zur qualitativen und quantitativen Bestimmung von Human-MIF in biologischen Flüssigkeiten und auf Zelloberflächen, die Verwendung der monoklonalen Antikörper zur Reinigung von Human-MIF, ferner pharmazeutische Präparate enthaltend gereinigten Human-MIF, seine Einzelproteine, monoklonale Antikörper gegen Human-MIF oder deren Derivate.

Hintergrund der Erfindung

Human-MIF aus menschlichen Zellen ist zwar als aktives Prinzip bekannt, hingegen bisher nur als Gemisch und zusammen mit anderen Proteinen in biologischen Flüssigkeiten beschrieben und von der Struktur her nicht charakterisiert. MIF gehört zur Gruppe der sogenannten Lymphokine, die biologisch aktive, lösliche Polypeptide umfasst, die von Lymphozyten und Monozyten oder Makrophagen ausgeschieden werden, wenn diese durch Antigene, Mitogene oder dergleichen stimuliert werden. Andere Beispiele von Lymphokinen sind Immun-Interferon ($\gamma$-Interferon), Interleukin 1 und 2 und Makrophagen-aktivierender Faktor (MAF). Diese Lymphokine regeln die Differenzierung, Aktivierung und Proliferation verschiedener Zelltypen des Immunsystems.

Nach dem bekannten Stand der Technik besteht Human-MIF aus einer Gruppe von Polypeptiden, die die Wanderungsfähigkeit von Makrophagen hemmen. Human-MIF wird nicht nur von aktivierten Lymphozyten, T- und B-Zellen, sondern auch von nicht-lymphoiden Zellen sezerniert, beispielsweise von wachsenden Fibroblasten und gewissen Tumorzellen. MIF lässt sich klar unterscheiden von $\gamma$-Interferon, Makrophagen-aktivierendem Faktor (MAF) und anderen Lymphokinen. Bisher war es aber nicht möglich, MIF aus menschlichen Zellen rein darzustellen und seine Struktur aufzuklären. Ueber Human-MIF war bekannt, dass er wahrscheinlich aus einem Gemisch von strukturell verwandten Polypeptiden mit einem Molekulargewicht von ungefähr 8,5, 18, 27, 36, 45 bzw. 67 kg/Mol (Kilo-Dalton) und einem isoelektrischen Punkt bei pH 5,1 und 2,9 besteht [G. Burmeister, H. Steffen, U. Feige und C. Sorg, Immunobiology 160, 15 (1981)].

Human-MIF spielt in der frühen Phase einer Entzündungsreaktion ("delayed type hypersensitivity reaction") eine entscheidende Rolle. Er induziert die Differenzierung von Monozyten und ruhenden Gewebe-Makrophagen zu funktionsbereiten entzündlichen Makrophagen. Gereinigter Human-MIF und seine Einzelproteine und monoklonale Antikörper, die spezifisch Human-MIF binden und seine Aktivität hemmen, sind deshalb von Bedeutung für die Diagnose und Therapie von Immunregulationskrankheiten und chronischen Entzündungskrankheiten. Monoklonale Antikörper, die Human-MIF binden und hemmen, sind wertvoll in der Bekämpfung von Kontaktekzemen, primärer chronischer Polyarthritis und verschiedenster Autoimmunkrankheiten. Gereinigter Human-MIF und seine Einzelproteine können die Infektresistenz, beispielsweise gegen Tuberkulose, Lepra oder Leishmaniose und gegen Candidose, sowie die Resistenz gegen Tumoren, insbesondere gegen Metastasen, erhöhen.

Die Verwendung von Antikörpern in Diagnose und Therapie war bis vor kurzem in ihrem Anwendungsbereich stark eingeschränkt. Antikörper wurden in geringen Mengen aus tierischem Serum als komplexe Mischung verschiedener Proteine gewonnen. Standardisierung der Antikörper war nicht möglich, weil jedes immunisierte tierische Individuum und auch ein einzelnes Individuum bei wiederholter Immunisierung jeweils ein Serum mit Antikörpern verschiedener Zusammensetzung ergibt. Durch Anwendung einer von Köhler und Milstein [G. Köhler und C. Milstein, Nature 256, 495 (1975)] entwickelten Technik ist es nun möglich geworden, Antikörper in homogener Form, d.h. sogenannte monoklonale Antikörper, reproduzierbar in industriellen Mengen aus Zellkulturen zu gewinnen. Durch Fusion von geeigneten Myeloma-Zellen mit Antikörper-produzierenden Lymphozyten aus einem mit Antigen immunisierten Spender entstehen Hybridoma-Zellen, die die Fähigkeit zur unbegrenzten Zellteilung und zu unbegrenztem Wachstum in vitro mit der Produktion eines homogenen Antikörpers verbinden. Somit ist es möglich, die Immunantwort eines Organismus auf ein bestimmtes Antigen zu verselbständigen und monoklonale Antikörper durch permanentes Kultivieren von Hybridoma-Zellen herzustellen.

Obwohl bis heute viele Beispiele für die Herstellung spezifischer Antikörper durch Hybridoma-Technik bekannt geworden sind und das allgemeine Vorgehen prinzipiell beschrieben ist, so treten doch bei jedem neuen Beispiel spezifische Probleme auf, die eine Anpassung der Technik an den gegebenen Fall

EP 0 162 812 B1

erfordern. Ohne solche Anpassung gibt es keine Gewissheit, dass die gewünschten Hybridoma-Zellen je gebildet werden, dass sie genetisch stabil sind, die gewünschten Antikörper herstellen und dass die dermassen hergestellten Antikörper die gewünschte Spezifität aufweisen. Der Grad des Erfolges wird im Prinzip beeinflusst durch Art und Reinheit des für die Immunisierung des Spenders verwendeten Antigens, die Technik der Zell-Fusion, das Vorgehen bei der Selektion geeigneter Hybridoma-Zellinien und die Art und Weise der Isolierung und Reinigung der Antikörper.

Eine wichtige Anwendung von Antikörpern, die die Verfügbarkeit von homogenen monoklonalen Antikörpern in grossen Mengen voraussetzt, wie sie jetzt durch die Hybridomatechnik ermöglicht wird, ist die Immunaffinitäts-Chromatographie. Dabei werden Antikörper mit einer gewünschten Spezifität auf ein festes Trägermaterial aufgebracht. Aus einer Lösung enthaltend eine Vielzahl verschiedener Verbindungen werden selektiv jene Verbindungen an die Antikörper und damit an das feste Trägermaterial gebunden, die ein Strukturelement (Determinante, Epitop) aufweisen, das vom Antikörper erkannt und gebunden wird. Nach Entfernung der Lösung mit den nicht erwünschten Verbindungen werden die erwünschten Verbindungen durch Auswaschen mit Reagentien, die die Bindung an die Antikörper aufbrechen, aus dem Trägermaterial herausgelöst und durch klassische Methoden isoliert.

Eine Aufgabe der vorliegenden Erfindung besteht darin, gereinigten Human-MIF und seine Einzelproteine zur Verfügung zu stellen. Diese Aufgabe wird mittels der erfindungsgemässen monoklonalen Antikörper gelöst.

Beschreibung der Erfindung

Die Erfindung betrifft gereinigten Human-Makrophagen-Migrationsinhibitonsfaktor (Human-MIF), der nur Proteine humanen Ursprungs enthält, die Epitope aufweisen, die vom monoklonalen Antikörper 1C5 gegen Human-MIF erkannt und gebunden werden, und seine Einzelproteine. Gereinigter Human-MIF besteht aus mindestens vier Einzelproteinen vom Molekulargewicht ca. 8, ca. 14, ca. 28 und ca. 45 kg/Mol. Gereinigter Human-MIF ist in Standard-Testanordnungen, bei denen die Wanderung von Makrophagen gemessen wird, aktiv.

Einzelproteine von gereinigtem Human-MIF sind Proteine, die nach den üblichen Methoden der Protein-Analyse, beispielsweise SDS-PAGE (Natriumdodecylsulfat-Polyacrylamid-Gel-Elektrophorese) oder Gel-Filtration-HPLC (Hochdruck-Flüssigchromatographie), homogen, die in Standard-Testanordnungen, bei denen die Wanderung von Makrophagen gemessen wird, aktiv und die Bestandteile von gereinigtem Human-MIF sind. Werden im Verlaufe der Trennung und Isolierung der Einzelproteine Detergentien oder andere denaturierende Reagentien zugegeben, so verändert sich die natürliche Tertiärstruktur der Proteine und damit die Eigenschaft, die Wanderung der Makrophagen zu hemmen, obwohl die Primärstruktur unverändert bleibt. Solche denaturierte Formen der Einzelproteine sind ebenfalls Gegenstand der Erfindung. Beispiele für Einzelproteine von gereinigtem Human-MIF sind die beiden Proteine mit dem Molekulargewicht von ca. 8 kg/Mol bzw. ca 14 kg/Mol und der N-terminalen Aminosäure-Sequenz

$$\text{Thr-Glu-Leu-Glu-Lys-Ala-Leu-Asn-Ser-Ile-Ile-Asp-Val-Tyr-His-Lys-Tyr-}X_1\text{-Leu-,}$$

worin die Bedeutung der Aminosäure $X_1$ nicht festgelegt ist, ferner Proteine mit dem Molekulargewicht von ca. 28 kg/Mol und ca. 45 kg/Mol.

In erster Linie betrifft die Erfindung das Einzelprotein von gereinigtem Human-MIF mit dem ungefähren Molekulargewicht von 8 kg/Mol und der N-terminalen Aminosäure-Sequenz

$$\text{Met-Leu-Thr-Glu-Leu-Glu-Lys-Ala-Leu-Asn-Ser-Ile-Ile-Asp-Val-Tyr-His-Lys-Tyr-Ser-}$$
$$\text{Leu-Ile-Lys-Gly-Asn-Phe-His-Ala-Val-Tyr-Arg-Asp-Asp-Leu-Lys-Lys-}$$
$$\text{Leu-Leu-Glu-Thr-Glu-}X_{42}\text{-Pro-Gln-Tyr-Ile-Arg-Lys-Lys-Gly-Ala-Asp-}$$
$$\text{Val-Trp-Phe-Lys-Glu-Leu-Asp-Ile-Asn-}X_{62}\text{-}X_{63}\text{-}X_{64}\text{-Ala-Val,}$$

4

worin die Bedeutung der Aminosäuren $X_{42}$ $X_{62}$, $X_{63}$ und $X_{64}$ nicht festgelegt ist, $X_{42}$ aber nur Ser oder Cys bedeuten kann.

Die Erfindung betrifft ferner ein Verfahren zur Herstellung von gereinigtem Human-MIF, der Proteine humanan Ursprungs enthält, die vom monoklonalen Antikörper 1C5 gegen Human-MIF erkannt und gebunden werden, und seiner Einzelproteine, dadurch gekennzeichnet, dass man eine Human-MIF enthaltende Lösung, z.B. einen Zellextrakt, Zellkulturüberstand oder ein Zellkulturfiltrat menschlicher Zellen, gewünschtenfalls nach an sich bekannten Reinigungsschritten,

a) mit einem Trägermaterial mit dem auf Human-MIF spezifischen monoklonalen Antikörper 1C5 in Kontakt bringt, ungebundene Proteine und andere Fremdstoffe entfernt, den an die Antikörper gebundenen Human-MIF selektiv abspaltet und isoliert und,

b) wenn erwünscht, den gereinigten Human-MIF in seine Einzelproteine auftrennt.

Lösungen, z.B. Zellextrakte, Zellkulturüberstände oder Zellkulturfiltrate menschlicher Zellen, die MIF enthalten, werden nach an sich bekannten Methoden zubereitet. Geeignete menschliche Zellen sind beispielsweise mononukleäre Zellen, die aus "buffy coat", der Schicht weisser Blutkörperchen, die sich bei der Zentrifugation von mit Citrat oder Heparin versetztem venösem Blut abscheidet, durch Leukapherese und/oder Zentrifugation in einem Dichtegradienten gewonnen werden können. Die mononukleären Zellen werden durch geeignete Hilfsstoffe, beispielsweise Concanavalin A oder Phytohämagglutinin, zur Produktion von MIF und anderen Lymphokinen angeregt und nach den üblichen Methoden während ca. 12 bis ca. 72 Stunden, bevorzugt zwischen 18 und 36 Stunden in geeigneten Kulturmedien, beispielsweise dem Medium RPMI 1640, denen gewünschtenfalls fötales Kälberserum, Pufferlösungen und/oder Antibiotica, wie Penicillin oder Streptomycin, zugesetzt werden, bei ca. 37°C und gewünschtenfalls $CO_2$-Begasung kultiviert. Lösungen enthaltend Human-MIF werden aus den Zellen und/oder den Zellkulturüberständen beispielsweise durch Extraktion, Filtration und/oder Zentrifugation gewonnen und gewünschtenfalls durch Zusatz von Antibiotica und/oder Protease-Inhibitoren stabilisiert. Solche Human-MIF-Lösungen können direkt mit dem an Trägermaterial gebundenen monoklonalen Antikörper 1C5 in Schritt a) in Kontakt gebracht werden, werden hingegen bevorzugt vorgängig mittels Ultrafiltration an Membranen mit Trenngrenzen von Molekulargewicht ca. 6 kg/Mol oder kleiner vorgereinigt, aufkonzentriert, gegebenenfalls dialysiert und gewünschtenfalls durch Chromatographie, z.B. an DEAE-Cellulose oder Sephadex®, weiter gereinigt.

In Schritt a) wird Human-MIF von anderen in den Lösungen enthaltenen Proteinen und Fremdstoffen abgetrennt, wobei die Trennwirkung beruhend auf bindenden Wechselwirkungen zwischen dem monoklonalen Antikörper 1C5 spezifisch auf Human-MIF und den erkannten antigenen Determinanten von Human-MIF genutzt werden. Dazu werden die Human-MIF enthaltenden Lösungen nach der an sich bekannten Methode der Immunaffinitäts-Chromatographie mit einem Trägermaterial, an welches der auf Human-MIF spezifische monoklonale Antikörper 1C5 gebunden ist, in Kontakt gebracht.

Ein geeignetes Trägermaterial auf anorganischer oder organischer Basis, beispielsweise Silikate, vernetzte Agarose, Dextran oder Polyacrylamid in geeignet funktionalisierter Form, wird auf an sich bekannte Weise, gegebenenfalls unter vorheriger Aktivierung, mit dem monoklonalen Antikörper 1C5 oder seinen Derivaten belegt. Beispielsweise wird ein Trägermaterial enthaltend aktivierte Esterfunktionen, z.B. N-Hydroxysuccinimidester-Gruppen, in einer wässrigen Puffer-Lösung suspendiert, mit einer Lösung des monoklonalen Antikörpers vermischt, anschliessend ungebundene monoklonale Antikörper aus-gewaschen und unbesetzte reaktive Stellen des Trägermaterials z.B. mit einem primären Amin, wie Aethanolamin, blockiert. Das Trägermaterial wird in einem geeigneten wässrigen Lösungsmittel, z.B. einer Salzlösung, wie NaCl-Lösung, oder einer Pufferlösung, wie Phosphat-gepufferter NaCl-Lösung, $NaHCO_3$-Lösung oder 3-(N-Morpholino)propansulfonsäure-Lösung, suspendiert und mit der Human-MIF enthaltenden Lösung in Kontakt gebracht, z.B. in eine Chromatographiesäule eingefüllt und die Human-MIF enthaltende Lösung aufgetragen und, gewünschtenfalls mit Druck, durch das Trägermaterial durchgepumpt. Ungebundene Proteine und andere Verunreinigungen werden mit wässrigen Lösungen, beispielsweise Puffer-Lösungen im pH-Bereich von ca. pH 5 bis ca. pH 9 und/oder Salz-Lösungen, z.B. NaCl-Lösung, weggewaschen. Der an den Antikörper auf dem Trägermaterial gebundene Human-MIF wird mit geeigneten wässrigen Lösungen, beispielsweise Puffer-Lösungen im pH-Bereich von ca. pH 2 bis ca. pH 5, wie Glycin-Puffer, oder pH-Gradienten verschiedener Zusammensetzung oder Salz-Lösungen, z.B. konzentrierter $NH_4SCN$-Lösung, eluiert. Die anfallenden, gereinigten Human-MIF enthaltenden Lösungen werden gegebenenfalls neutralisiert und daraus nach an sich bekannten Methoden, beispielsweise durch Chromatographie über Sephadex®, Elektrodialyse, elektrophoretische Konzentration und/oder Vakuumzentrifugation, der gereinigte Human-MIF isoliert.

Gewünschtenfalls wird in Schritt b) der gereinigte Human-MIF in seine Einzelproteine aufgetrennt, beispielsweise indem man nach an sich bekannten Methoden das Proteingemisch chromatographisch in Fraktionen verschiedenen Molekulargewichts trennt. Beispielsweise wird der gereinigte Human-MIF durch präparative Natriumdodecylsulfat-Polyacrylamid-Gel-Elektrophorese (SDS-PAGE) aufgetrennt, homogene Molekulargewichtsfraktionen aus dem Gel eluiert und, z.B. durch Chromatographie über Sephadex®, elektrophoretische Konzentration und/oder Vakuumzentrifugation, in reiner Form isoliert. Der gereinigte Human-MIF kann auch durch präparative Gel-Filtration-HPLC in homogene Molekulargewichtsfraktionen aufgetrennt und daraus die Einzelproteine isoliert werden.

Die Erfindung betrifft ferner neue monoklonale Antikörper gegen Human-Makrophagen-Migrationsinhibitionsfaktor (Human-MIF), sowie Derivate davon.

Die erfindungsgemässen monoklonalen Antikörper binden und/oder hemmen die biologische Aktivität von Human-MIF. Die Bindung von monoklonalen Antikörpern an Human-MIF wird mit Vorteil in einem Immunoassay bestimmt, beispielsweise mit einer Testanordnung, bei der Human-MIF auf einen festen Träger aufgebracht, der beschichtete Träger mit einer monoklonalen Antikörper-Lösung inkubiert und dabei gebundene monoklonale Antikörper mit einem Zweit-Antikörper, der eine Radioisotop- oder Enzymmarkierung trägt, entwickelt werden. An Human-MIF gebundene monoklonale Antikörper werden so durch Messung der Radioaktivität bzw. durch eine Enzym-Substrat-Reaktion bestimmt. Geeignet ist beispielsweise auch eine Testanordnung, bei der die monoklonalen Antikörper auf einen Träger fixiert, mit einer Human-MIF-haltigen Lösung inkubiert und schliesslich der Restgehalt an Human-MIF-Aktivität dieser Lösung bestimmt wird.

Die Human-MIF-Aktivität einer Lösung wird in an sich bekannter Weise durch Bestimmung der Hemmwirkung auf die Wanderung von geeignet aktivierten menschlichen Makrophagen gemessen. Beispielsweise kann eine Versuchsanordnung gewählt werden, bei der die Wanderungsstrecke von in Agarosetropfen auf Titerplatten aufgebrachten Makrophagen in einer Probelösung gemessen wird.

Bevorzugt sind monoklonale Antikörper gegen Human-MIF, die von Maus/Maus-, Ratte/Ratte- oder Ratte/Maus-Hybridoma-Zellen produziert werden. Beispiele für solche bevorzugte erfindungsgemässe monoklonale Antikörper sind der monoklonale Antikörper der Subklasse $IgG_1 x$ mit der Bezeichnung 1C5, der von der Hybridoma-Zellinie 1C5 produziert wird, und der monoklonale Antikörper der Subklasse $IgG_{2a}$ mit der Bezeichnung 7D10, der von der Hybridoma-Zellinie 7D10 produziert wird. Die monoklonalen Antikörper 1C5 und 7D10 binden an Human-MIF, ohne seine biologische Aktivität zu hemmen.

Erfindungsgemässe Derivate von monoklonalen Antikörpern sind beispielsweise Bruchstücke, wie Fab, Fab' oder $F(ab')_2$-Fragmente, die ihre Spezifität für die antigenen Determinanten von Human-MIF beibehalten, radioaktiv markierte monoklonale Antikörper, die beispielsweise mit radioaktivem Iod ($^{125}I$, $^{131}I$), Kohlenstoff ($^{14}C$), Schwefel ($^{35}S$), Tritium ($^3H$) oder dergleichen markiert sind, monoklonale Antikörper-Konjugate mit Biotin oder Avidin oder monoklonale Antikörper-Konjugate mit Enzymen, wie Meerrettich-Peroxidase, alkalischer Phosphatase, $\beta$-D-Galactosidase, Glucose-Oxidase, Glucoamylase, Carboanhydrase, Acetylcholinesterase, Lysozym, Malat-Dehydrogenase oder Glucose-6-phosphat-Dehydrogenase. Bevorzugte Derivate sind mit $^{125}$Iod markierte monoklonale Antikörper und Antikörper-Konjugate mit Biotin.

Die Erfindung betrifft auch an sich bekannte Verfahren zur Herstellung der monoklonalen Antikörper gegen Human-MIF und ihrer Derivate, gekennzeichnet dadurch, dass man besagte monoklonale Antikörper produzierende Hybridoma-Zellen

a) in vitro kultiviert und aus den Kulturüberständen die monoklonalen Antikörper isoliert, oder

b) in vivo in einem geeigneten Säugetier vermehrt und aus den Körperflüssigkeiten dieses Säugetiers die monoklonalen Antikörper isoliert, und,

c) wenn erwünscht, einen erhaltenen monoklonalen Antikörper in ein Derivat davon überführt.

Geeignete Kulturmedien für die in vitro Kultivierung nach Verfahren a) sind die üblichen Standard-Kulturmedien, beispielsweise Dulbecco's Modified Eagles-Medium oder RPMI 1640-Medium, gegebenenfalls ergänzt mit fötalem Kälber-Serum. Zur Isolierung der monoklonalen Antikörper werden die Proteine in den Kulturüberständen mit Ammoniumsulfat oder dergleichen gefällt und mit den üblichen chromatographischen Methoden, beispielsweise Gel-Filtration, Ionenaustauscher-Chromatographie, Chromatographie an DEAE-Cellulose oder Immunaffinitäts-Chromatographie, gereinigt.

Grosse Mengen der gewünschten Antikörper können erhalten werden durch Vermehrung der Hybridoma-Zellen in vivo gemäss Verfahren b). Dazu werden Zellklone in Säugetiere, bevorzugt in syngene Säugetiere injiziert und nach 1-3 Wochen die monoklonalen Antikörper aus den Körperflüssigkeiten dieser Säuger isoliert. Beispielsweise werden Hybridoma-Zellen abstammend von Balb/c-Mäusen intraperitoneal in gegebenenfalls mit einem Kohlenwasserstoff wie Pristan vorbehandelte Balb/c-Mäuse injiziert und nach 8-10 Tagen diesen Tieren Aszitesflüssigkeit entnommen. Die gewünschten monoklonalen Antikörper werden aus den Körperflüssigkeiten nach an sich bekannten Methoden isoliert, beispielsweise durch Fällung mit Ammoniumsulfat oder dergleichen und chromatographische Reinigung, z.B. über DEAE-Cellulose, Hydroxylapatit (HPHT, high performance hydroxylapatite column chromatography), Ionenaustauscherharz, durch Gel-Filtration oder Immunaffinitäts-Chromatographie.

Erfindungsgemässe Bruchstücke von monoklonalen Antikörpern, beispielsweise Fab, Fab' oder F(ab')$_2$-Fragmente, die ihre Spezifität für die antigenen Determinanten von Human-MIF beibehalten, werden nach an sich bekannten Methoden hergestellt, beispielsweise durch Behandlung von nach Verfahren a) oder b) hergestellten monoklonalen Antikörpern mit Enzymen wie Pepsin oder Papain und/oder Spaltung von Disulfid-Bindungen durch chemische Reduktion.

Mit Iod ($^{125}$I, $^{131}$I) radioaktiv markierte monoklonale Antikörper werden aus den erfindungsgemässen monoklonalen Antikörpern durch an sich bekannte Iodierung, beispielsweise mit radioaktivem Natrium- oder Kaliumiodid und einem chemischen Oxidationsmittel, wie Natriumhypochlorit, Chloramin T oder dergleichen, oder einem enzymatischen Oxidationsmittel, wie Lactoperoxidase, Glucoseoxidase und Glucose, erhalten. Erfindungsgemässe radioaktiv markierte monoklonale Antikörper werden auch hergestellt, indem man auf an sich bekannte Weise den Kulturmedien für die in vitro-Kultivierung radioaktiv markierte Nährstoffe enthaltend radioaktiven Kohlenstoff ($^{14}$C), Tritium ($^3$H), Schwefel ($^{35}$S) oder dergleichen, beispielsweise L-($^{14}$C)-Leucin, L-($^3$H)-Leucin oder L-($^{35}$S)-Methionin, zugibt und die monoklonalen Antikörper nach Verfahren a) gewinnt.

Enzym-markierte erfindungsgemässe monoklonale Antikörper erhält man nach an sich bekannten Methoden, indem man nach Verfahren a) oder b) hergestellte monoklonale Antikörper und das gewünschte Enzym mit einem Kupplungsreagens, beispielsweise Glutaraldehyd, Periodat, N,N'-o-Phenylendimaleimid, N-(m-Maleimidobenzoyloxy)-succinimid, N-(3-(2'-Pyridyldithio)-propionoxy)-succinimid oder dergleichen, umsetzt. Gleichermassen erhält man Konjugate von erfindungsgemässen monoklonalen Antikörpern mit Avidin. Konjugate mit Biotin erhält man nach an sich bekannten Methoden, indem man erfindungsgemässe monoklonale Antikörper beispielsweise mit Biotin-N-hydroxysuccinimidylester umsetzt.

Die Erfindung betrifft ferner Hybridoma-Zellinien, dadurch gekennzeichnet, dass sie monoklonale Antikörper gegen Human-Makrophagen-Migrationsinhibitionsfaktor (Human-MIF) produzieren.

Bevorzugt sind monoklonale, gegen Human-MIF gerichtete Antikörper produzierende Hybridoma-Zellinien, die Hybride von Maus-Myeloma-Zellinien und Maus- oder Ratten-Lymphozyten sind.

Ganz besonders bevorzugt ist die Hybridoma-Zellinie mit der Bezeichnung 1C5, die am 13.7.1984 in der "Collection Nationale de Cultures de Microorganismes" des Institut Pasteur in Paris unter der Nummer I-316 hinterlegt worden ist. Die Zellinie 1C5 ist ein Hybrid der Maus-Myeloma-Zellinie P3-X63-Ag8.653 und eines B-Lymphozyten der Milz einer Balb/c-Maus. Gleichermassen bevorzugt ist die Hybridoma-Zellinie mit der Bezeichnung 7D10, die am 29.1.1985 in der "Collection Nationale de Cultures de Microorganismes" des Institut Pasteur in Paris unter der Nummer I-418 hinterlegt worden ist. Die Zellinie 7D10 ist ein Hybrid der Maus-Myeloma-Zellinie P3-X63-Ag8.653 und eines B-Lymphozyten der Milz einer DA-Ratte. Beide Zellinien sind genetisch stabil, scheiden monoklonale Antikörper gleichbleibender Spezifität aus und können aus tiefgefrorenen Kulturen durch Auftauen und Re-Klonierung aktiviert werden.

Die Erfindung betrifft ferner an sich bekannte Verfahren zur Herstellung von Hybridoma-Zellen, die monoklonale Antikörper gegen Human-MIF produzieren, dadurch gekennzeichnet, dass man geeignete Säugetiere mit MIF oder MIF-Konjugaten immunisiert, dem Säugetier entnommene Antikörper-produzierende Zellen mit Myeloma-Zellen fusioniert, die erhaltenen Hybridoma-Zellen kloniert und diejenigen Zellklone, die die gewünschten monoklonalen Antikörper produzieren, selektioniert.

Als Antigene können irgendwelche MIF-haltige Protein-Fraktionen oder MIF-Konjugate verwendet werden, z.B. aus menschlichen mononukleären Zellen wie oben beschrieben gewonnene MIF-haltige Protein-Fraktionen oder Konjugate dieser Protein-Fraktionen mit geeigneten immunogenen Trägern, beispielsweise Proteinen, Polysacchariden, Latex-Partikeln oder Zellen. Voraussetzung für die Verwendung von tierischem, z.B. murinem, MIF enthaltenden Protein-Fraktionen ist, dass der betreffende tierische MIF und Human-MIF identische Epitope aufweisen. Konjugate werden nach an sich bekannten Methoden hergestellt, beispielsweise durch Kopplung mit Carbodiimiden, Periodat, Glutaraldehyd, N,N'-o-Phenylendimaleimid, N-(m-Maleimidobenzoyloxy)-succinimid, N-(3-(2'-Pyridyldithio)-propionoxy)-succinimid oder dergleichen. Bevorzugt werden zur Immunisierung Konjugate von mit Glutaraldehyd vorbehandelten Schaf-Erythrozyten und MIF-haltigen Protein-Fraktionen aus menschlichen oder aus Mäuse-Zellen eingesetzt.

Bevorzugte Säugetiere zur Immunisierung mit Human-MIF sind Mäuse oder Ratten, insbesondere Balb/c-Mäuse. Zur Immunisierung mit Maus-MIF werden bevorzugt Ratten, z.B. DA-Ratten, verwendet. Die Immunisierungen werden in an sich bekannter Weise vorgenommen, beispielsweise indem man drei bis acht Injektionen parenteral, z.B. intraperitoneal und subcutan mit antigenen Human-MIF-Konjugaten in Abständen von ein bis zehn Tagen verabreicht, gewünschtenfalls zusammen mit einem die Lymphozyten-Produktion anregenden Hilfsstoff, beispielsweise komplettem oder inkomplettem Freund'schen Adjuvans. Es ist auch möglich, die Tiere durch zwei bis vier Injektionen vorzuimmunisieren und erst nach einer Pause von 8 bis 12 Monaten weitere Injektionen zu verabreichen.

Antikörper-produzierende Zellen der immunisierten Tiere, vorzugsweise Milzzellen, werden zwei bis sechs Tage nach der letzten Immunisierung den Tieren entnommen und mit Myeloma-Zellen einer geeigneten Zellinie in Gegenwart eines Fusions-Promotors fusioniert. Mehrere verschiedene Myeloma-Zellinien und daraus abgeleitete Zellinien sind bekannt als geeignete Fusionspartner. Bevorzugt sind Myeloma-Zellen, denen das Enzym Hypoxanthin-Guanin-Phosphoribosyl-Transferase(HGPRT) oder das Enzym Thymidin-Kinase (TK) fehlt und die deshalb in einem selektiven Kulturmedium, das Hypoxanthin, Aminopterin und Thymidin (HAT-Medium) oder Hypoxanthin und Azaserin enthält, nicht überleben. Besonders bevorzugt sind Myeloma-Zellen und daraus hergestellte Zellinien, die in HAT-Medium oder Hypoxanthin/Azaserin-Medium nicht überleben und keine Immunglobuline oder Teile davon ausscheiden, beispielsweise die Zellinien X63-Ag8.653 und Sp2/O-Ag14. Die von Balb/c-Mäusen abstammende Zellinie X63-Ag8.653 ist nicht nur geeignet zur Fusion mit Lymphozyten von Mäusen, z.B. Balb/c Mäusen, sondern auch mit Lymphozyten von Ratten, wie DA-Ratten.

Als Fusions-Promoter kommen Sendai-Virus oder andere Paramyxoviren, gegebenenfalls in UV-inaktivierter Form, Kalzium-Ionen, oberflächenaktive Lipide, wie Lysolezithin, oder Polyäthylenglykol in Frage. Bevorzugt werden Myeloma-Zellen mit einem zwei- bis zehnfachen Ueberschuss an Milzzellen aus immunisierten Tieren in einer Lösung enthaltend etwa 30 bis etwa 50 % Polyäthylenglykol eines Molekulargewichts zwischen etwa 1000 und etwa 6000 fusioniert.

Nach der Fusion werden die Zellen aufgeteilt und in selektivem HAT-Medium oder Hypoxanthin/Azaserin-Medium kultiviert, wobei nur Hybridoma-Zellen überleben, weil diese von den Myeloma-Zellen die Fähigkeit zum Wachstum in vitro und von Antikörper-produzierenden Zellen der immunisierten Tiere die fehlenden HGPRT- oder TK-Gene und damit die Fähigkeit zum Ueberleben in selektivem Medium vereinen.

Geeignete Kulturmedien für die Expansion der Hybridoma-Zellen sind die üblichen Standard-Kulturmedien, beispielsweise Dulbecco's Modified Eagles-Medium oder RPMI 1640-Medium. Vorzugsweise werden zu Beginn des Zellwachstums sogenannte Feederzellen zugegeben, beispielsweise normale peritoneale Mäuse-Exsudatzellen, Milzzellen, Knochenmark-Makrophagen oder dergleichen. In regelmässigen Intervallen werden besagte Kulturmedien durch selektives HAT-Medium oder Hypoxanthin/Azaserin-Medium ergänzt, um ein Ueberwachsen der Hybridoma-Zellen durch gewöhnliche Myeloma-Zellen zu verhindern.

Die Zellkultur-Ueberstände der Hybridoma-Zellen werden daraufhin untersucht, ob sie die gewünschten monoklonalen Antikörper enthalten. Bevorzugt wird dazu ein Radioimmunoassay, ein Enzymimmunoassay und/oder eine Bestimmung der MIF-Aktivität verwendet, wie sie oben beschrieben sind. Die solchermassen ausgewählten Zellklone werden in den üblichen Standardmedien kultiviert und gewünschtenfalls auf an sich bekannte Weise tiefgefroren und/oder durch begrenzende Verdünnung oder durch Austragen auf Agar rekloniert.

Die Erfindung betrifft im weiteren die Verwendung der monoklonalen Antikörper gegen Human-Makrophagen-Migrationsinhibitionsfaktor (Human-MIF) und ihrer Derivate zur qualitativen und quantitativen Bestimmung von Human-MIF, insbesondere in biologischen Flüssigkeiten und auf Zelloberflächen. Beispielsweise können die erfindungsgemässen monoklonalen Antikörper in irgendeinem der an sich bekannten Immunoassays verwendet werden, die die bindenden Wechselwirkungen zwischen Antigen (Human-MIF) und monoklonalem Antikörper nutzen. Beispiele für solche Assays sind Radioimmunoassays (RIA), Enzym-Immunoassays, Immuno-Fluoreszenz-Tests, Latex-Agglutination oder Hämagglutination.

Die erfindungsgemässen Antikörper können als solche oder als radioaktiv markierte Derivate gegebenenfalls in Kombination mit andern markierten Antikörpern und/oder Proteinen in einem Radioimmunoassay (RIA) eingesetzt werden. Irgendeine der bekannten Abwandlungen eines RIA kann verwendet werden, beispielsweise RIA in homogener Phase, Festphasen- oder heterogener RIA, einfacher RIA oder doppelter (Sandwich) RIA mit direkter oder indirekter (kompetitiver) Bestimmung von Human-MIF.

Bevorzugt ist ein RIA, bei dem ein geeigneter Träger, beispielsweise die Plastik-Oberfläche einer Titerplatte oder eines Test-Röhrchens, z.B. aus Polystyrol, Polypropylen oder Polyvinylchlorid, Glas- oder Plastik-Perlen, Filterpapier, Dextran-, Celluloseacetat- oder Nitrocellulose-Blätter oder dergleichen, mit einer Human-MIF-haltigen Probe- oder Standard-Lösung durch einfache Adsorption oder gegebenenfalls nach Aktivierung des Trägers, beispielsweise mit Glutaraldehyd oder Bromcyan, beschichtet und mit einer Lösung eines erfindungsgemässen monoklonalen Antikörpers und dann mit einer Lösung eines Zweitantikörpers inkubiert wird, wobei der Zweitantikörper, beispielsweise ein Kaninchen-anti-Maus-Immunglobulin, den erfindungsgemässen monoklonalen Antikörper erkennt und bindet. Die Menge gebundenen Zweitantikörpers wird durch Messung der gebundenen Radioaktivität bestimmt, entweder direkt, wenn der Zweitantikörper radioaktiv markiert ist, oder nach Entwicklung mit einem radioaktiv markierten Protein mit hoher Affinität gegenüber diesem Zweitantikörper, beispielsweise [125]I-markiertem Protein A aus Staphylococcus aureus.

8

Die erfindungsgemässen monoklonalen Antikörper können als solche oder als Enzym-markierte Derivate in einem Enzym-Immunoassay eingesetzt werden. Solche Immunoassays sind beispielsweise Test-Anordnungen, bei denen an sich bekannte Enzym-markierte Antikörper, die Epitope der erfindungsgemässen monoklonalen Antikörper erkennen und binden, oder Enzym-markierte erfindungsgemässe monoklonale Antikörper-Derivate eingesetzt werden. Anstelle von Enzym-markierten Antikörpern können auch Antikörper-Biotin-Konjugate zusammen mit Avidin-Enzym-Konjugaten eingesetzt werden. Beispiele für in Enzym-Immunoassays eingesetzte Enzyme sind Meerrettich-Peroxidase, alkalische Phosphatase, $\beta$-D-Galactosidase, Glucose-Oxidase, Glucoamylase, Carboanhydrase, Acetylcholinesterase, Lysozym, Malat-Dehydrogenase oder Glucose-6-phosphat-Dehydrogenase.

Bevorzugt ist ein ELISA-Test (enzyme-linked immunosorbent assay), in welchem man einen Träger, wie er oben für einen einfachen RIA-Test beschrieben ist und der gewünschtenfalls mit Erythrozyten belegt ist, entweder mit einer Human-MIF-haltigen Probe- oder Standard-Lösung durch einfache Adsorption oder gewünschtenfalls nach Aktivierung des Trägers bzw. der Träger-gebundenen Erythrozyten mit Glutaraldehyd beschichtet oder mit auf Human-MIF zu untersuchenden Zellen belegt, anschliessend diesen Träger mit einer Lösung eines erfindungsgemässen monoklonalen Antikörpers und schliesslich mit einer Lösung eines Enzym-markierten Zweitantikörpers, der den erfindungsgemässen monoklonalen Antikörper erkennt und bindet, inkubiert. Dabei wird die Menge des gebundenen Zweitantikörpers, beispielsweise eines Peroxidasemarkierten Kaninchen-anti-Maus-Immunglobulins, durch Entwicklung mit Enzym-Substrat sichtbar gemacht und bestimmt.

Besonders bevorzugt ist ein ELISA-Test (enzyme-linked immunosorbent assay), in welchem man einen Träger, wie er oben für einen einfachen RIA-Test beschrieben ist, mit einer Lösung eines erfindungsgemässen monoklonalen Antikörpers durch einfache Adsorption oder gegebenenfalls nach Aktivierung des Trägers, beispielsweise mit Glutaraldehyd oder Bromcyan, beschichtet, anschliessend diesen Träger mit einer Human-MIF-haltigen Probe- oder Standard-Lösung und schliesslich entweder mit einer Lösung eines Enzym-markierten erfindungsgemässen Antikörpers, der gegebenenfalls ein anderes Epitop von Human-MIF erkennt, oder bevorzugt mit einer Lösung eines mit Biotin konjugierten erfindungsgemässen Antikörpers gefolgt von einer Lösung eines Avidin-Enzym-Konjugats inkubiert. Dabei wird die Menge des gebundenen Enzym- oder Biotin-markierten Antikörpers durch Entwicklung mit Enzym-Substrat sichtbar gemacht und bestimmt.

Bevorzugte Enzyme in den erfindungsgemässen Enzym-Immunoassays sind Meerrettich-Peroxidase, die beispielsweise mit den Enzym-Substraten 5-Aminosalicylsäure, o-Phenylendiamin, 3,3'-Dimethoxybenzidin, 3,3',5,5'-Tetramethylbenzidin, 2,2'-Azino-bis-(3-äthylbenzthiazolin-6-sulfonsäure) oder dergleichen und Wasserstoffperoxid entwickelt werden kann, und alkalische Phosphatase, die beispielsweise aus dem Enzym-Substrat p-Nitrophenylphosphat p-Nitrophenol freisetzt.

Die erfindungsgemässe Verwendung von monoklonalen Antikörpern gegen Human-MIF und ihrer Derivate zur qualitativen und quantitativen Bestimmung von Human-MIF umfasst auch andere an sich bekannte Immunoassays, beispielsweise Immunofluoreszenz-Tests unter Verwendung von Antikörper- oder Antigen-Konjugaten mit fluoreszierenden Stoffen, Latex-Agglutination mit Antikörper- oder Antigen-beschichteten Latex-Partikeln oder Hämagglutination mit Antikörper- oder Antigen-beschichteten roten Blutkörperchen oder dergleichen.

Die beschriebenen Immunoassays können verwendet werden zur Bestimmung der in biologischen Flüssigkeiten, insbesondere in menschlichem Blut, oder auf Zelloberflächen in fixierter Form vorhandenen Menge Human-MIF und erleichtern damit die Diagnose von durch Immunregulationsstörungen verursachten Krankheiten. Beispielsweise kann bei geringer Infektionsresistenz eine ursächliche Immunregulationsstörung diagnostiziert werden, wenn im Blut keine oder unterdurchschnittliche Mengen von Human-MIF gefunden werden. Ferner kann die Bestimmung von Human-MIF in pathologischen Geweben, z.B. in Melanomen, Granulomen und Hyperplasien, eine einfache Diagnose ermöglichen, weil MIF nur in bestimmten Gewebetypen und nur in bestimmten pathologischen Zuständen vorkommt (Tabelle).

**Tabelle:** Bindung des monoklonalen Antikörpers 1C5 an gefrorene Gewebeschnitte

| | |
|---|---|
| **Normale Gewebe:** | |
| A) Leber | − |
| B) Milz | + (nur wenige Zellen) |
| C) Haut | + (nur wenige Makrophagen) |
| D) Lunge | − |
| **Pathologische Gewebe:** | |
| E) Primäre Melanoma (viel Zell-Infiltrate) | ++ (Zell-Infiltrate) |
| Melanoma-Metastasen (wenig Zell-Infiltrate) | + (nur wenige Zellen) |
| Melanoma-Mikrometastasen (in Lymphknoten) | − |
| F) BCG-Granuloma (1, 3 oder 5 Wochen nach der Impfung) | + |
| G) Sézary-Syndrom | + |
| H) Syringolymphoide Hyperplasie | + |
| I) Subakutes Ekzem | − |
| J) Hypereosinophiles Syndrom | − |

+ Bindung nachgewiesen, − keine Bindung nachgewiesen

Die Erfindung betrifft auch Test-Kits zur qualitativen und quantitativen Bestimmung von Human-Makrophagen-Migrationsinhibitionsfaktor (Human-MIF), dadurch gekennzeichnet, dass sie monoklonale Antikörper gegen Human-MIF und/oder Derivate davon und gegebenenfalls Hilfsmittel enthalten.

Erfindungsgemässe Test-Kits für einen Radioimmunoassay enthalten beispielsweise einen geeigneten Träger, gegebenenfalls gefriergetrocknete oder konzentrierte Lösungen eines erfindungsgemässen Antikörpers oder eines radioaktiv markierten erfindungsgemässen Antikörper-Derivats, Lösungen eines gegebenenfalls radioaktiv markierten Zweitantikörpers und/oder gegebenenfalls Lösungen von radioaktiv markiertem Protein A aus Staphylococcus aureus, Standardlösungen von gereinigtem Human-MIF oder seiner Einzelproteine, Puffer-Lösungen, Fixier-Lösungen enthaltend Glutaraldehyd, Detergentien zur Verhinderung von unspezifischer Adsorption und Aggregatbildung, Pipetten, Reaktionsgefässe, Eichkurven und dergleichen.

Erfindungsgemässe Test-Kits für einen Enzym-Immunoassay enthalten beispielsweise einen geeigneten Träger, gegebenenfalls gefriergetrocknete oder konzentrierte Lösungen eines erfindungsgemässen monoklonalen Antikörpers, eines Enzym-markierten oder eines Biotinmarkierten erfindungsgemässen Antikörper-Derivats, gegebenenfalls gefriergetrocknete oder konzentrierte Lösungen eines Enzym-markierten Antikörpers, der die erfindungsgemässen monoklonalen Antikörper erkennt und bindet, gegebenenfalls gefriergetrocknete oder konzentrierte Lösungen von Avidin-Enzym-Konjugaten, Enzym-Substrate in fester oder gelöster Form, Standardlösungen von gereinigtem Human-MIF- oder seiner Einzelproteine, Puffer-Lösungen, Fixier-Lösungen enthaltend Glutaraldehyd, Detergentien, Pipetten, Reaktionsgefässe, Eichkurven und dergleichen.

Die Erfindung betrifft im weiteren die Verwendung der monoklonalen Antikörper gegen Human-Makrophagen-Migrationsinhibitionsfaktor (Human-MIF) und ihrer Derivate zur Reinigung von Human-MIF. Beispielsweise kann Human-MIF mit an sich bekannten Trennmethoden, deren Trennwirkung auf bindenden Wechselwirkungen zwischen monoklonalem Antikörper und antigenen Determinanten von Human-MIF beruhen, getrennt werden. Eine bevorzugte Trennmethode ist die Immunaffinitäts-Chromatographie, wie sie oben beschrieben ist.

Die Erfindung betrifft im weiteren pharmazeutische Präparate enthaltend eine therapeutisch wirksame Menge von gereinigtem Human-MIF, seiner Einzelproteine, von monoklonalen Antikörper gegen Human-MIF oder von Derivaten solcher monoklonalen Antikörper und eine signifikante Menge eines pharmazeutischen Hilfsmittels. Geeignete Derivate von monoklonalen Antikörpern sind Bruchstücke, z.B. Fab, Fab' oder F(ab')-$_2$-Fragmente, die ihre Spezifität für die antigenen Determinanten von Human-MIF beibehalten haben.

Bei den erfindungsgemässen pharmazeutischen Präparaten handelt es sich um solche zur enteralen, z.B. nasalen, rektalen oder oralen, sowie bevorzugt zur parenteralen, z.B. intramuskulären, subcutanen oder intravenösen, Verabreichung an Warmblüter, z.B. den Menschen. Je nach beabsichtigter Methode der Verabreichung können die pharmazeutischen Präparate in Dosiseinheitsform, z.B. in Ampullen, Vials, Suppositorien, Dragées, Tabletten, Kapseln oder Nasalsprays in flüssiger oder fester Form vorliegen.

Die zu verabreichende Menge der therapeutisch wirksamen Verbindungen hängt vom Zustand des Warmblüters, z.B. des Menschen ab, wie dem Körpergewicht, der Art und Schwere der Erkrankung und dem Allgemeinzustand, ferner von der Art der Verabreichung, und erfolgt gemäss der Einschätzung des behandelnden Arztes. Die wirksame Dosis für Human-MIF und seine aktiven Einzelproteine liegt in der Grössenordnung von 0,001-1 $\mu$g pro kg Körpergewicht und Tag, für monoklonale Antikörper gegen Human-MIF und deren Derivate in der Grössenordnung von 0,001-1 mg pro kg Körpergewicht und Tag.

Die erfindungsgemässen pharmazeutischen Präparate enthalten die üblichen anorganischen oder organischen, festen oder flüssigen pharmazeutisch verwendbaren Trägerstoffe, gegebenenfalls zusammen mit anderen therapeutisch wirksamen Verbindungen und/oder Hilfsstoffen. Vorzugsweise verwendet man Lösungen oder Suspensionen des Wirkstoffes, insbesondere isotonische wässrige Lösungen oder Suspensionen, ferner auch lyophilisierte Präparate, welche kurz vor Gebrauch in Wasser gelöst werden. Die pharmazeutischen Präparate können sterilisiert sein und/oder Konservierstoffe, Stabilisiermittel, Netzmittel, Emulgiermittel, Löslichkeitsvermittler, viskositäts-erhöhende Stoffe, Salze zur Regulierung des osmotischen Drucks und/oder Puffer, ferner auch andere Proteine, z.B. Human-Serum-Albumin oder menschliche Blutplasma-Präparate, enthalten.

Bevorzugt sind pharmazeutische Präparate in Form von Liposomen in wässriger Dispersion enthaltend eine therapeutisch wirksame Menge von gereinigtem Human-MIF oder seiner Einzelproteine. Geeignet sind insbesondere Liposomen mit einer Population von möglichst homogener Grösse und einem Durchmesser von ca. $2,0 \times 10^{-8}$ bis $5,0 \times 10^{-6}$ m bestehend aus einer oder mehreren Doppelschichten von Lipidkomponenten, beispielsweise amphipatischen Lipiden, z.B. Phospholipiden, wie Lecithin, Kephalin oder Phosphatidsäure, und gegebenenfalls neutralen Lipiden, z.B. Cholesterin, umschliessend einen wässrigen Innenraum mit erfindungsgemässem gereinigtem Human-MIF oder seinen Einzelproteinen. Bevorzugt sind Liposomen aus einem Gemisch von synthetischem Phosphatidylserin und Phosphatidylcholin.

Die nachfolgenden Beispiele illustrieren die Erfindung, schränken jedoch deren Umfang in keiner Weise ein.

Die in den Beispielen verwendeten Abkürzungen haben folgende Bedeutung:

| | |
|---|---|
| ELISA | Enzym-Assay (enzyme-linked immunosorbent assay) |
| HAT | Hypoxanthin/Aminopterin/Thymidin |
| HPLC | Hochdruck-Flüssigchromatographie (high pressure liquid chromatography) |
| HT | Hypoxanthin/Thymidin |
| Min. | Minute(n) |
| MIF | Makrophagen-Migrationsinhibitionsfaktor |
| PBS | Phosphat-gepufferte physiologische Kochsalzlösung |
| RIA | Radioimmuno-Assay |
| SDS | Natriumdodecylsulfat (sodium dodecyl sulfate) |
| SDS-PAGE | SDS-Polyacrylamid-Gel-Elektrophorese |
| SRBC | Schaf-Erythrozyten |
| Std. | Stunde(n) |
| Tris | Tris-(hydroxymethyl)-aminomethan |
| upm | Umdrehungen pro Minute |

Beispiel 1: Gewinnung einer Protein-Fraktion enthaltend Human-MIF

Mononukleäre Zellen werden aus "buffy coat", der Schicht weisser Blutkörperchen, die sich bei der Zentrifugation von mit Citrat oder Heparin versetztem venösem Blut abscheidet, gewonnen und gereinigt durch eine Zwei-Schritt-Methode bestehend aus Leukapherese und kontinuierlicher Zentrifugation über einen Ficoll®-Gradienten im IBM Blut-Zellseparator (IBM 2997) [U. Feige und C. Sorg, J. Immunol. Methods 66, 161 (1984)]. Die mononukleären Zellen werden in Spinner-Medium (Seromed) gewaschen, dann in einer Konzentration von $5 \times 10^6$ Zellen in 0,17 ml RPMI 1640 Medium pro cm$^2$ Kulturgefässboden für 2 Std. mit 0,67 $\mu$g Concanavalin A pro $10^6$ Zellen stimuliert. Erneuerung des Mediums RPMI 1640 und anschliessende 20stündige Inkubation bei 37°C und 5 % $CO_2$-Begasung liefert den Lymphokine enthaltenden Kulturüberstand. Dieser wird 30 Min. bei 17'000 upm (SS34 Rotor, Sorvall-Zentrifuge) bei 4°C zentrifugiert. Der zellfreie Ueberstand wird über Sephadex® G25, das in 0,05M $NH_4HCO_3$ äquilibriert ist, entsalzt und die Protein enthaltenden Fraktionen anschliessend lyophilisiert. Das Lyophilisat wird in 0,01M Natriumphosphat-Puffer pH 7,5, dem 0,1M NaCl zugesetzt ist, aufgenommen und im gleichen Puffer über Sephadex® G100 chromatographiert. Die Human-MIF enthaltenden Fraktionen umfassend Proteine im Molekulargewichtsbereich 8 bis 14 kg/Mol (Kilo-Dalton) werden vereinigt und durch Einengen an einem Rotavapor 16fach aufkonzentriert.

Beispiel 2: Herstellung der Hybridoma-Zellen

2.1 Konjugation von Human-MIF enthaltenden Protein-Fraktionen an Schaf-Erythrozyten

2,5 ml gepackte Schaf-Erythrozyten (sheep red blood cells, SRBC, Behringwerke) werden in 20 ml PBS suspendiert. 100 $\mu$l dieser SRBC-Suspension werden mit 900 $\mu$l einer Glutaraldehyd-Lösung in PBS während 5 Min. inkubiert, so dass die Endkonzentration des Glutaraldehyds 0,05 % beträgt. Die derart vorbehandelten SRBC werden zweimal mit eiskaltem dest. Wasser gewaschen, abzentrifugiert und anschliessend während 1 Std. bei 20°C mit 300 $\mu$l der Human-MIF enthaltenden Fraktionen aus Beispiel 1 inkubiert. Diese Suspension wird zur Immunisierung eingesetzt.

2.2 Immunisierung

Balb/c-Mäuse werden durch 3 Injektionen von je 0,5 ml der mit Human-MIF gekoppelten SRBC-Suspension aus Beispiel 2.1 zusammen mit 0,5 ml komplettem Freund'schen Adjuvans am Tag 0, 7 und 10 immunisiert. Dabei wird eine Hälfte der Injektionsmenge intraperitoneal (i.p.), die andere Hälfte in vier Portionen subcutan (s.c.) injiziert. Zwei weitere ("booster") Injektionen von je 0,5 ml Konjugat-Suspension ohne Adjuvans werden am Tag 13 und 14 i.p. verabreicht. Die Milz der behandelten Mäuse wird am Tag 18 entnommen.

2.3 Zellfusion

Entsprechend dem Verfahren von Köhler und Milstein [G. Köhler und C. Milstein, Nature 256, 495 (1975)) werden $10^8$ Milz-Lymphozyten mit 3,3 x $10^7$ Maus-Myelomazellen P3-X63-Ag8.653 [J.F. Kearney, A. Radbruch, B. Liesegang und R. Rajewsky, J. Immunol. 123, 1548 (1979)] in 1,5 ml einer Lösung von 35 % Polyäthylenglykol 4000 (Merck, Darmstadt) und 9,7 % Dimethylsulfoxid in Dulbecco's Modified Eagle Medium vermischt. Nach der Fusion werden die Zellen in 600 Löchern von Falcon 3040 96-Loch-Platten ausplattiert und in Littlefields HAT-Medium (Hypoxanthin/Aminopterin/Thymidin-Standardmedium) [J.W. Littlefield, Science 145, 709 (1974)] zusammen mit Knochenmark-Makrophagen als Feeder-Zellen kultiviert. Nach 10 Tagen in HAT-Medium wird in RPMI 1640 HT-Medium weiterkultiviert.

Beispiel 3: Prüfung der Hybridoma-Zellen auf Antikörper-Spezifität

3.1 γ-Globulin-Nachweis

Die Ueberstände der Hybridoma-Zellkulturen werden in einem ELISA (enzyme-linked immunosorbent assay) geprüft, bei dem ein mit Peroxidase konjugierter Zweitantikörper aus Kaninchen, der Maus-γ-Globulin erkennt und bindet, eingesetzt wird. Von 103 Hybridoma-Zellinien produzieren 72 Klone γ-Globuline.

3.2 Spezifität auf Proteine im gewünschten Molekulargewichts-Bereich

Syngene Maus-Erythrozyten (1 x $10^6$ Erythrozyten in 100 $\mu$l PBS pro Loch) werden auf mit poly-L-Lysin (25 mg/ml) beschichtete 96-Loch-Platten (Dynatech Microtiter) aufgebracht und über Nacht bei 4°C inkubiert. Nicht gebundene Erythrozyten werden durch mehrmaliges Waschen mit PBS entfernt. Die so gebundenen Erythrozyten werden wie in Beispiel 2.1 beschrieben in Glutaraldehyd anfixiert, gewaschen und anschliessend mit den Human-MIF enthaltenden Fraktionen im Molekulargewichts-Bereich 8 bis 14 kg/Mol (Beispiel 1) inkubiert. Nicht gebundenes Protein wird durch mehrmaliges Waschen entfernt und die Platten in PBS enthaltend 0,1 % $NaN_3$ gelagert. Die Platten werden mit Kulturüberstand der Hybridoma-Zellen, anschliessend mit einem mit alkalischer Phosphatase gekoppelten Kaninchen-anti-Maus-Immunglobulin-Zweitantikörper inkubiert und mit p-Nitrophenylphosphat in 2-Amino-2-äthyl-1,3-propandiol-Puffer (Serva) entwickelt. Das freigesetzte p-Nitrophenol wird photometrisch bei 405 nm nachgewiesen. Durch den beschriebenen ELISA werden monoklonale Antikörper erkannt, die an die Erythrozyten-gekoppelten Proteine der Human-MIF enthaltenden Molekulargewichtsfraktionen aus Beispiel 1 binden. Von den 72 Zellklonen, die $\gamma$-Globuline ausscheiden, produzieren 15 Klone monoklonale Antikörper, die in diesem Assay gebunden werden.

### 3.3 Spezifität auf Human-MIF

$\gamma$-Globuline aus den Kulturüberständen der gemäss Beispiel 3.2 identifizierten Klone werden mit Ammoniumsulfat bei 50 % Sättigung gefällt, die Fällungen in PBS aufgenommen und nach einer Vorschrift des Herstellers an Affi-Gel® 10 (Bio-Rad) gekoppelt. Die so immobilisierten $\gamma$-Globuline werden mit Human-MIF enthaltenden Ueberständen von mit Concanavalin A stimulierten mononukleären Zellen (Beispiel 1) über Nacht bei 4°C inkubiert und anschliessend bei 3000 upm und 4°C 10 Min. in einer IEC-Zentrifuge zentrifugiert. Die überstehende Lösung wird anschliessend auf ihren Rest-Gehalt an Human-MIF im MIF-Test gemäss Beispiel 4 überprüft. Auf diese Weise wird eine Zellinie mit der Bezeichnung 1C5 identifiziert, die monoklonale Antikörper gegen Human-MIF produziert.

### Beispiel 4: Test auf Human-Makrophagen-Migrationsinhibitionsfaktor (MIF-Test)

### 4.1 Herstellung des Percoll-Gradienten

9 Teile Percoll® der Dichte 1,007 (Pharmacia), 1 Teil 10-fach konzentriertes MEM Earl's Medium (Seromed) und 10 Teile Spinner-Medium (Seromed) werden 12 Min. bei 12'000 upm in einer Sorvall Zentrifuge (DuPont) bei 20°C vermischt.

### 4.2 Zielzellgewinnung für den MIF-Test

"Buffy coat", d.h. Zellkonzentrat weisser Blutkörperchen, von 9-12 Spendern wird mit Spinner-Medium (Seromed) im Verhältnis 1:2 verdünnt, auf 20°C erwärmt und auf Ficoll paque® (Pharmacia) nach Vorschrift des Herstellers bei 20°C aufgetrennt. Die mononukleären Zellen der Interphase werden nach der Zentrifugation zweimal mit dem gleichen Medium gewaschen, dann in einem nach Beispiel 4.1 hergestellten Percoll-Gradienten durch Zentrifugation in 40 Min. bei 20°C und 1600 upm in einer IEC-Zentrifuge in Monozyten und Lymphozyten aufgetrennt. Die Monozyten werden dreimal mit Spinner-Medium gewaschen, in McCoy's Medium (Seromed), dem 20 % Pferdeserum zugesetzt ist, aufgenommen und über Nacht bei 37°C und 7 % $CO_2$-Begasung im selben Medium in Teflon-Säckchen kultiviert.

### 4.3 Durchführung des MIF-Tests

Die kultivierten Monozyten aus Beispiel 4.2 werden zweimal in MEM-Dulbecco Medium (Seromed) gewaschen und in einem Gemisch aus einem Teil doppelt-konzentriertem MEM-Dulbecco Medium und einem Teil 0,4%iger Agarose (Miles) so aufgenommen, dass die Zellkonzentration 5 x $10^5$ Zellen pro ml beträgt. Diese Zellsuspension wird in Tropfen zu 1 $\mu$l mittels einer Hamilton-Spritze in die inneren 60 Löcher einer 96-Loch-Platte (Falcon, Microtest III) pipettiert. Nach 15 Min. bei 4°C ist die Agarose erstarrt. Anschliessend werden 100 $\mu$l der zu testenden Probelösungen pro Loch aufgegeben. Als Kontroll-Lösung wird MEM-Dulbecco Medium, dem 1 % Pferdeserum zugesetzt ist, verwendet. Verdünnungen der zu testenden Probelösungen werden im selben Medium hergestellt. Inkubiert werden die Platten für 15 Std. bei 37°C in einer feuchten Atmosphäre und 7 % $CO_2$-Begasung.

Gemessen wird die Wanderung der Monozyten aus den Agarose-Tropfen mit Hilfe eines skalierten Fadenkreuzes im Okular eines Mikroskopes. Die Messung wird so durchgeführt, dass man die eine Achse des Fadenkreuzes tangential an den Tropfenrand legt und mit der senkrecht dazu stehenden Achse die Strecke vom Tropfenrand bis zur Wanderungsgrenze der Zellen bestimmt. Die Wanderung der Zellen in der Kontroll-Lösung, gemessen in Skalenteilen, wird als 100 % Wanderung bzw. 0 % Wanderungshemmung festgelegt. Darauf bezogen wird die in den Probelösungen erreichte Wanderungsstrecke als % Hemmung der Wanderung ausgedrückt. Die biologische Aktivität einer Probelösung wird in MIF-Einheiten ausgedrückt. Eine MIF-Einheit ist definiert als jene biologische Aktivität, die in der beschriebenen Test-Anordnung 30 % Wanderungshemmung hervorruft. Die Anzahl MIF-Einheiten einer Probelösung entspricht demnach dem Faktor, um den diese Lösung verdünnt werden muss, um gerade 30 % Wanderungshemmung hervorzurufen.

Beispiel 5: Isolierung und Reinigung der monoklonalen Antikörper aus Aszites

Balb/c Mäuse werden intraperitoneal mit 0,4 ml Pristan (Carl Roth) vorbehandelt. Nach einer Woche werden 2 bis 5 x $10^6$ klonierte Hybridoma-Zellen intraperitoneal injiziert. Aszitische Flüssigkeit wird von jeder Maus wiederholt genommen und bei -80°C gefroren. Die gesammelte Flüssigkeit wird aufgetaut und 30 Min. bei 4°C mit 16'000 upm zentrifugiert. Das Fett wird abgesaugt und zum verbleibenden Debrisfreien Ueberstand langsam unter Rühren bei 0°C eine gesättigte Ammoniumsulfatlösung zugetropft, bis eine Konzentration von 50 % erreicht ist. Die derart ausgefällte rohe Immunglobulin-Fraktion wird mit 0,1M Tris-HCl (pH 8,2) über DEAE Affi-Gel Blue® (Bio-Rad) gemäss den Angaben des Herstellers chromatographiert. Aktive Fraktionen werden vereinigt und mit Amicon XM50-Filter (Amicon) konzentriert.

Beispiel 6: Herstellung einer Antikörper-Säule

Affi-Gel® 10 (Bio-Rad) wird nach den Angaben des Herstellers mit kaltem destilliertem Wasser und Kupplungspuffer pH 7,5 (MOPS, 3-(N-Morpholino)propansulfonsäure) gewaschen. Eine 50 %ige Suspension des Gels in Kupplungspuffer (1 ml) wird in ein Plastikrohr übertragen, mit der gleichen Menge gereinigter Antikörperlösung (20 mg monoklonale Antikörper 1C5) vermischt und 4 Std. bei Zimmertemperatur rotiert. Danach wird das Gel mit Kupplungspuffer gewaschen. Zur Blockierung der noch freien aktiven Stellen wird das Gel mit 0,1 ml 1M Aethanolamin-HCl (pH 8,0) pro ml Gel während 2 Std. bei Raumtemperatur behandelt, dann mit PBS enthaltend 10 mM Natriumazid pro ml Gel gewaschen und darin bei 4°C gehalten. Der Kupplungsgrad wird durch Messung der Extinktion bei 280 nm bestimmt und beträgt 12 bis 30 mg monoklonaler Antikörper pro ml Gel.

Beispiel 7: Isolierung und Reinigung von Human-MIF-Proteinen

7.1 Produktion von Human-MIF

$10^{11}$ mononukleäre Zellen werden nach dem in Beispiel 1 beschriebenen Verfahren aus "buffy coat" isoliert und mit Concanavalin A (ConA) zur Lymphokinproduktion stimuliert. Die stimulierten Zellen werden in Nunc-Wannenstapeln mit einer Kulturfläche von je 6000 cm$^2$ bei einer Zellkonzentration von 5 x $10^6$ Zellen in 0,25 ml Medium RPMI 1640 pro cm$^2$ Oberfläche bei 37°C und 5 % $CO_2$-Begasung 24 Std. kultiviert. Danach wird der Zellkulturüberstand 30 Min. bei 35000 g und 4°C zentrifugiert. Der klare Ueberstand wird mit Phenylmethansulfonylfluorid, einem Serinprotease-Inhibitor, (Endkonzentration: 50 $\mu$Mol/1) und mit Natriumazid (Endkonzentration: 0,05 %) versetzt.

7.2 Konzentration des Zellkulturüberstandes

Die Zellkulturüberstände aus Beispiel 7.1 werden mittels Ultrafiltration in einer Amicon Rührzelle über eine YMS-Membran (nominelle Trenngrenze: Molekulargewicht 5 kg/Mol) 15-fach konzentriert. Um Verluste durch Adsorption zu vermeiden und einer möglichen Aggregation der Proteine entgegenzuwirken, wird die Ultrafiltration unter Zugabe von Triton X-100® (Alkylphenylpolyäthylenglykol,Rohm & Haas) durchgeführt; die Endkonzentration an Triton X-100® beträgt ca. 0,2 % (w/v). Das Konzentrat wird bei 35000 g zentrifugiert und durch einen 0,25 $\mu$m Filter (Millipore) filtriert.

7.3 Immunaffinitätschromatographie

1 1 des Lymphokin-Konzentrates aus Beispiel 7.2 wird bei 4°C mit einer Flussrate von ca. 10 ml/Std. durch eine Antikörpersäule (Beispiel 6) enthaltend 4 ml Gel mit einem Kupplungsgrad von 12 mg Antikörper pro ml Gel gepumpt. Unspezifisch gebundene Proteine und Begleitsubstanzen werden durch Waschen mit 100 ml PBS/ 0,5 M NaCl/ 0,2 % Triton X-100®/ 0,02 % Natriumazid, pH 7,3, dann 20 ml PBS und schliesslich 10 ml 0,1 M NaCl bei einer Flussrate von 15 ml/Std. aus der Säule eluiert. Die spezifisch gebundenen Human-MIF-Proteine werden mit einer Lösung von 0,1 M Glycinhydrochlorid/ 0,1 M NaCl, pH 2,6, eluiert, wobei die Elution durch automatische Messung der Absorption bei 280 nm (Uvicord S, LKB Instruments) verfolgt wird. Um Verluste durch Adsorption zu vermeiden, wird das Eluat fraktionsweise (je 3 ml) in Polypropylen-Röhrchen, die je 100 $\mu$l einer 3 % SDS-Lösung enthalten, aufgefangen. Die Protein enthaltenden Fraktionen werden vereinigt und durch Zugabe von 1 M Tris-Lösung neutralisiert.

## 7.4 Elektrodialyse, elektrophoretische Konzentration

Das neutralisierte Eluat aus Beispiel 7.3 wird mittels eines "ISCO Electrophoretic Concentrators" Model 1750 (Isco. Inc.) und einer Spectrapor®-Membran (Spectrum Medical Industries) mit einer nominellen Trenngrenze Molekulargewicht 3,3 kg/Mol gegen 25 mM Ammoniumacetat/0,01 % SDS, pH 8,3 dialysiert und gleichzeitig auf ein Volumen von 0,2 ml konzentriert. Das dialysierte Konzentrat wird zur Entfernung des Ammoniumacetats in einer Vakuumzentrifuge (Speed Vac Concentrator, Savant Inc.) zur Trockne eingedampft.

## 7.5 Analyse der MIF-Proteine durch SDS-Polyacrylamidgel-Elektrophorese (SDS-PAGE)

Ein Aliquot (ca. 2 %) des dialysierten Konzentrates (Beispiel 7.4) wird nach der Methode von Laemmli [U.K. Laemmli, Nature 227, 680-685 (1970)] auf 15 % Polyacrylamid-Flachgel einer Elektrophorese unterworfen. Die Proteinbanden werden durch Anfärbung mit Comassie Brillantblau (Fluka) und mit der Silberfärbungsmethode nach C.A. Merril et al. [Anal. Biochem. 110, 201 (1981)] sichtbar gemacht. Das von der Antikörpersäule eluierte Material enthält danach Proteine mit Molekulargewicht ca. 8 kg/Mol und ca. 14 kg/Mol, ferner vergleichsweise geringe Mengen mit Molekulargewicht ca. 28 kg/Mol und ca. 45 kg/Mol.

## 7.6 Charakterisierung der MIF-Aktivität der durch Immunaffinitäts-Chromatographie isolierten Proteine

Eine verlustarme präparative Auftrennung und Isolierung der Einzelproteine von Human-MIF geschieht vorteilhafterweise in Gegenwart eines Detergens, z.B. von SDS (siehe Beispiele 7.3, 7.4 und 7.7). Die MIF-Aktivität der Proteine wird durch die Wechselwirkung mit SDS zerstört. Die MIF-Aktivität wird den einzelnen Molekulargewichtsfraktionen mit Hilfe von biosynthetisch radioaktiv markierten Lymphokinen wie folgt zugeordnet: $10^8$ humane mononukleäre Zellen werden in 5 ml RPMI 1640 Medium, das 3 $\mu$g Concanavalin A pro ml enthält, suspendiert und in einem Zellkulturgefäss mit 25 cm$^2$ Oberfläche (Nuncolon® TC 25) 2 Std. bei 37°C unter 5 % $CO_2$-Begasung inkubiert. Anschliessend wird das Medium entfernt und die Zellen in Leucin-freiem RPMI 1640 Medium, dem 10 $\mu$Ci L-[U-$^{14}$C]Leucin (Amersham) pro ml zugefügt wird, 20 Std. kultiviert. Der radioaktive Zellkulturüberstand wird mit 500 ml nichtmarkiertem, Human-MIF enthaltendem Zellkulturüberstand (Beispiel 7.1) vereinigt und, wie in Beispiel 7.2 beschrieben, 15-fach konzentriert. Analog Beispiel 7.3 werden die Human-MIF-Proteine durch Immunaffinitäts-Chromatographie mit einer Säule enthaltend 0,5 ml Gel isoliert, wobei das Eluat ohne SDS-Zusatz aufgefangen wird. Nach Neutralisation mit 1 M Tris-Lösung wird das Eluat durch Chromatographie über eine Sephadex® G 25 Säule (Pharmacia) in 50 mM Ammoniumbicarbonat-Lösung überführt und lyophilisiert. Der Rückstand wird in 0,5 ml PBS gelöst und bei ca. 35000 g zentrifugiert. Ein Aliquot von 0,1 ml wird mittels HPLC auf einer Bio-Sil® TSK-125 Säule (7,5 x 300 mm, BioRad) fraktioniert. Die Fraktionen werden durch Messung der Radioaktivität (als Mass für markierte Proteine) und durch Bestimmung der MIF-Aktivität (Beispiel 4) charakterisiert. Die Radioaktivität und MIF-Aktivität befinden sich in den gleichen Fraktionen, deren Position Molekulargewichtsbereichen von ca. 8 kg/Mol bzw. ca. 14 kg/Mol entsprechen. Ein weiteres Aliquot des radioaktiven Eluats wird analog Beispiel 7.5 einer SDS-PAGE unterworfen. Die radioaktiven Proteine werden durch Autoradiographie sichtbar gemacht. Starke Banden erscheinen in Molekulargewichtsbereichen von ca. 8 kg/Mol und ca. 14 kg/Mol, schwächere Banden in den Bereichen von ca. 28 kg/Mol und ca. 45 kg/Mol.

## 7.7 Präparative Trennung der Human-MIF-Proteine durch SDS-PAGE und Isolierung durch Elektroelution

Nach Beispiel 7.4 aufbereitetes Material aus ca. 50 1 Zellkulturüberständen wird durch SDS-PAGE in einem diskontinuierlichen Puffersystem nach der Methode von Laemmli [Nature 227, 680 (1970)] in einem vertikalen Slab-Gel-Elektrophorese-System aufgetrennt. Die Probe wird in 300 $\mu$l einer Pufferlösung der Zusammensetzung 0,05 M Tris•HCl/ 3 % (w/v) SDS/0.02 M Dithiothreitol/ 10 % (v/v) Glycerin, pH 6,8, aufgelöst und in einer Breite von 1,5 cm auf das 1,5 mm dicke Gel enthaltend 15 % Acrylamid aufgetragen. Um einer möglichen Derivatisierung der Proteine durch freie Radikale und Oxidantien im Gel vorzubeugen, wird dem Kathodenpuffer Natriumthioglycolat in einer Konzentration von 0,1 mM zugegeben [siehe M.W. Hunkapiller et ei., Methode in Enzymology 91, 227 (1983)]. Zur Sichtbarmachung der Proteine wird das Gel für 5 Min. in eine eiskalte 0,25 M KCl-Lösung gelegt [siehe D.A. Hager und R.R. Burgess, Anal Biochem. 109, 76 (1980)]. Sichtbare Banden in der Molekulargewichtsregion von 8 Kg/Mol und 14 Kg/Mol werden herausgeschnitten und die Proteine mit einer von Bhown et al. [Anal. Biochem 103, 184 (1980)] beschriebenen Technik aus dem Gel eluiert. Hierzu werden in einem "ISCO Electrophoretic Concentrator", Model 1750 (Isco Inc.) mit einer Spectropor®-Membran (Spectrum Medical Industries, nominelle Trenngrenze 3,5 kg/Mol) die Proteine bei einer Leistung von 2 Watt mit 0,05 M Ammoniumacetat/ 0,01 % SDS in 8 Std. eluiert. Die eluierten Proteine befinden sich im "sampling cup" in einem Volumen von ca. 150 $\mu$l und sind frei von Puffersubstanzen (Glycin, Tris). Zur Entfernung des Ammoniumacetates werden die Elektroeluate in einer Vakuumzentrifuge (Speed Vac Concentrator, Savant Inc.) zur Trockene eingedampft.

Die Homogenität der eluierten Proteine und die Ausbeuten werden überprüft, indem jeweils ca. 5 % der Eluate einer analytischen SDS-PAGE unterzogen werden (Beispiel 7.5), wobei parallel zu den Proben definierte Mengen von Proteinen mit bekannten Molekulargewichten der Elektrophorese unterworfen werden. SDS-PAGE zeigt im Eluat aus der Molekulargewichtregion 8 kg/Mol eine homogene Proteinbande und im Eluat aus der Molekulargewichtsregion 14 kg/Mol eine homogene Bande.

## 7.8 Präparative Trennung und Isolierung der Human-MIF-Proteine durch Gel-Filtration-HPLC

2 l eines Konzentrates von Human-MIF enthaltenden Kulturüberständen aus Beispiel 7.2 werden mit einer Flussrate von ca. 15 ml/Std. durch eine Antikörpersäule (Beispiel 6) enthaltend 5 ml Gel mit einem Kupplungsgrad von ca. 12 mg 1C5 Antikörper pro ml Gel gepumpt. Die Säule wird analog Beispiel 7.3 gewaschen und die spezifisch gebundenen Human-MIF-Proteine mit 0,1 M Glycinhydrochlorid / 0,1 M NaCl, pH 2,6, eluiert, wobei das Eluat fraktionsweise in Polypropylen-Röhrchen ohne Zusatz von SDS aufgefangen wird. Die Elution wird durch Messung der Absorption bei 280 nm und durch Bestimmung der MIF-Aktivität (Beispiel 4) verfolgt.

Die vereinigten Eluate aus 4 Ansätzen (aus insgesamt 8 l Kulturüberstand-Konzentrat) werden analog Beispiel 7.4 gegen 0,005 M Anmoniumacetat, pH 7,5, dialysiert und gleichzeitig auf ein Volumen von 0,2 ml Konzentriert. Das Konzentrat wird auf eine mit 0,05 M Ammoniumacetat, pH 7,5, äquilibrierte HPLC-Säule (9,5 mm x 500 mm) enthaltend Si 300 Polyol® 0,003 mm (Serva, Heidelberg, BRD) aufgetragen und bei einem Druck von 40 bar und einer Durchflussgeschwindigkeit von 0,3 ml/Min. mit 0,05 M Ammoniumacetat, pH 7,5, eluiert. Die Elution wird durch Messung der Absorption bei 280 nm, durch Bestimmung der MIF-Aktivität (Beispiel 4) und durch den Enzym-Immunoassay von Beispiel 10 verfolgt. Die Hauptanteile an Proteingehalt und MIF-Aktivität finden sich in Fraktionen, deren Position Molekulargewichtsbereichen von ca. 8 kg/Mol bzw. ca. 14 kg/Mol entspricht; geringere Anteile werden in Fraktionen der Molekulargewichtsbereiche von ca. 28 kg/Mol und ca. 45 kg/Mol bestimmt. Zur Entfernung des Ammoniumacetats werden die Human-MIF-Proteine enthaltenden Fraktionen wiederholt lyophilisiert.

## Beispiel 8: Aminosäure-Sequenzanalyse

### 8.1 MIF-Protein mit Molekulargewicht 8 kg/Mol

Das gereinigte Protein mit Molekulargewicht 8 kg/Mol aus Beispiel 7.7 wird mit einem "Gas-phase Protein Sequencer Model 470" (Applied Biosystems) nach der von M.W. Hunkapiller und L.E. Hood [Methods in Enzymology 91, 399 (1983)] beschriebenen Methode sequenziert. Die Umlagerung der Anilinothiazolinon-Derivate zu Phenylthiohydantoin-(PTH)-Aminosäuren erfolgt durch Behandlung mit 25 % Trifluoressigsäure bei 50°C.

Die PTH-Aminosäuren werden auf einer Zorbax CN®-Säule (Du Pont, 200 x 4,6 mm) [siehe R. Knecht et al., Anal. Biochem. 130, 65 (1983)] analysiert. Folgende eindeutige N-terminale Aminosäuresequenz wird ermittelt:

```
            5                    10                        15
Met-Leu-Thr-Glu-Leu-Glu-Lys-Ala-Leu-Asn-Ser-Ile-Ile-Asp-Val-
          20                   25                        30
Tyr-His-Lys-Tyr-Ser-Leu-Ile-Lys-Gly-Asn-Phe-His-Ala-Val-Tyr-
          35                   40                        45
Arg-Asp-Asp-Leu-Lys-Lys-Leu-Leu-Glu-Thr-Glu-X₄₂-Pro-Gln-Tyr-
          50                   55                        60
Ile-Arg-Lys-Lys-Gly-Ala-Asp-Val-Trp-Phe-Lys-Glu-Leu-Asp-Ile-
          65
Asn-X₆₂-X₆₃-X₆₄-Ala-Val.
```

$X_{42}$ bedeutet Ser oder Cys. $x_{62}$, $x_{63}$ und $X_{64}$ stellen nicht bestimmte Aminosäuren dar.

## 8.2 MIF-Protein mit Molekulargewicht 14 kg/Mol

Das gereinigte Protein mit Molekulargewicht 14 kg/Mol aus Beispiel 7.7 wird analog Beispiel 8.1 sequenziert. Folgende eindeutige N-terminale Aminosäuresequenz wird ermittelt:

$$X_1-Leu-Thr-Glu-\overset{5}{Leu}-Glu-Lys-Ala-Leu-\overset{10}{Asn}-Ser-Ile-Ile-Asp-\overset{15}{Val}-Tyr-$$

$$His-Lys-Tyr$$

$X_1$ stellt eine nicht bestimmte Aminosäure dar.

### Beispiel 9: Herstellung von monoklonalen Antikörpern gegen Maus-MIF

#### 9.1 Gewinnung von Protein-Fraktionen enthaltend Maus-MIF

Nach dem von C. Sorg [Molecular Immunology 17, 565 (1980)] beschriebenen Verfahren wird durch Stimulierung von Milzzellen aus 100 Balb/c-Mäusen mit Concanavalin A ein Zellüberstand erhalten, der nach Chromstographie an Sephadex® G 100 ca. 50 ml einer Lösung ergibt, die Maus-MIF-Proteine des Molekulargewichtsbereiches 40 bis 70 kg/Mol enthält.

#### 9.2 Konjugation von Maus-MIF enthaltenden Protein-Fraktionen an Schaf-Erythrozyten

Die Fraktionen aus Beispiel 9.1 werden auf ein Gesamtvolumen von 4,5 ml eingeengt und analog Beispiel 2.1 an mit Glutaraldehyd vorbehandelte Schaf-Erythrozyten (SRBC) gekoppelt.

#### 9.3 Immunisierung von Ratten

DA-Ratten werden durch sieben Injektionen von je 0,5 ml der mit Maus-MIF gekoppelten SRBC-Suspension aus Beispiel 9.2 zusammen mit 0,5 ml kompletten Freund'schen Adjuvans am Tage 0, 10, 43 und 309 immunisiert. Dabei wird eine Hälfte der Injektionsmenge intraperitoneal (i.p.), die andere Hälfte in vier Portionen subcutan (s.c.) injiziert. Weitere ("booster") Injektionen von je 0,5 ml Konjugatsuspension ohne Adjuvans werden am Tage 312, 313 und 314 i.p. verabreicht. Die Milz der behandelten Ratten wird am Tage 317 entnommen.

#### 9.4 Zellfusion

Analog Beispiel 2.3 werden $1,14 \times 10^8$ Milz-Lmphozyten immunisierter Ratten mit $3,5 \times 10^7$ Maus-Myleomazellen P3-X63-Ag8.653 fusioniert und in HAT-Medium kultiviert.

#### 9.5 Selektion von Hybridoma-Zellen, die Antikörper gewünschter Spezifität ausscheiden

Analog dem in Beispiel 3.1 und 3.2 beschriebenen Verfahren werden aus den insgesamt 244 nach Beispiel 9.4 gewonnenen Hybridoma-Zellinien 49 Klone ausgewählt, die monoklonale Antikörper ausscheiden, welche an mit Ratten-Erythrozyten gekoppelte Proteine (Kopplung analog Beispiel 9.2) aus Fraktionen enthaltend Maus-MIF binden. γ-Globuline aus den Kulturüberständen dieser 49 Zellklone werden nach dem Verfahren von Beispiel 3.3 an Affi-Gel® 10 (Bio-Rad) gekoppelt und mit Maus-MIF enthaltenden Ueberständen von mit Concanavalin A stimulierten Milz-Zellen (Beispiel 9.1) inkubiert. Die überstehende Lösung wird anschliessend auf ihren Rest-Gehalt an Maus-MIF geprüft. Der Gehalt an Maus-MIF wird dazu in einem analog Beispiel 4.3 vorgenommenen MIF-Test bestimmt, wobei anstelle der kultivierten Human-Monozyten jedoch peritoneale Maus-Makrophagen eingesetzt und diese in MEM-Dulbecco Medium enthaltend 10 % FCS aufgenommen und in Agarase-Tropfen fixiert werden.

Mit diesem Selektionsverfahren werden 6 Hybridoma-Zellinien, u.a. der Zellklon mit der Bezeichnung 7D10 identifiziert, die monoklonale Antikörper gegen Maus-MIF produzieren. Diese Antikörper binden ebenfalls Human-MIF in einem MIF-Test gemäss Beispiel 4, zeigen also Kreuzaktivität.

9.6 Isolierung und Reinigung der monoklonalen Antikörper aus Aszites

Analog Beispiel 5 werden mit Pristan vorbehandelten Balb/c-Mäusen (nu/nu) 1 x $10^7$ klonierte Hybridoma-Zellen intraperitoneal injiziert und die Antikörper aus Aszites-Flüssigkeit gewonnen.

Beispiel 10: Enzym-Immunoassay zur Bestimmung von Human-MIF (MIF-ELISA)

Je 100 $\mu$l einer gereinigten Antikörperlösung 1C5 (anti-Human-MIF) einer Konzentration von 10 $\mu$g/ml werden in den Löchern einer 96-Loch-Mikrotiterplatte (Costar; Technorama) 1 Std. bei 37°C inkubiert. Die Platten werden dreimal mit PGT-20-Puffer (PBS, dem 0,2 % Gelatine (Merck) und 0,05 % Tween 20 zugesetzt sind) gewaschen und noch vorhandene Protein-reaktive Bindungsstellen des Plattenbodens durch Inkubation mit je 250 $\mu$l PGT-20-Puffer pro Loch während 1 Std. bei 37°C abgesättigt. Je 50 $\mu$l von Verdünnungsreihen einer Probelösung oder einer Standardlösung enthaltend Human-MIF werden in den Vertiefungen der Mikrotiterplatten 1 Std. bei 37°C inkubiert. Ungebundene Anteile werden dreimal mit PGT-20-Puffer weggewaschen und dann je 50 $\mu$l einer Lösung eines aus Antikörper 7D10 (anti-Maus-MIF, kreuzreaktiv mit Human-MIF) und Biotin-N-Hydroxysuccinimidyl-esters (Medac, Mol-Verhältnis Biotin zu Antikörper 7D10 = 4 - 7 : 1) in PBS hergestellten und über Sephadex® G 25 gereinigten Konjugats (10 $\mu$g/ml) in den Vertiefungen 30 Min. bei 37°C inkubiert. Nach dreimaligem Waschen der Platte mit PGT-20-Puffer werden die auf die Platte gebundenen Biotin-Antikörper-Konjugate durch Inkubation (30 Min. bei 37°C) mit je 100 $\mu$l einer 1:3000-fach verdünnten Lösung eines Avidin-Meerrettich-Peroxidase-Konjugats (0,3 $\mu$g/ml) (Sigma) umgesetzt. Die aufgenommene Menge Enzym wird nach Waschen der Platte durch Entwicklung (30 Min. bei 37°C) mit einer Lösung von 2,2'-Azino-bis-(3-äthylbenzothiazolin-6-sulfonsäure)-diammoniumsalz (ABTS, Boehringer Mannheim, 55 mg in 100 ml Citrat/Phosphat-Buffer, 0,05 M Citrat, 0,1 M $Na_2HPO_4$, pH 4,0, enthaltend 16 $\mu$l 30 % $H_2O_2$) und einer photometrischen Messung bei 405 nm bestimmt.

Anstelle des Biotin-Antikörper 7D10-Konjugats kann auch ein analog hergestelltes Biotin-Antikörper 1C5-Konjugat eingesetzt werden, doch weist ein solchermassen duchgeführter ELISA eine geringere Empfindlichkeit für Human-MIF auf.

Beispiel 11: Test-Kit für MIF-ELISA

Ein Test-Kit für den in Beispiel 10 beschriebenen Enzym-Immunoassay enthält:

Mikrotiterplatten aus Polypropylen

20 ml Lösung des monoklonalen Antikörpers 1C5 (10 $\mu$g/ml)
10 ml Lösung eines Biotin-Konjugats des monoklonalen Antikörpers 7D10 (Mol-Verhältnis Biotin zu Antikörper 7D10 = 5 : 1, 10 $\mu$g/ml)
1 ml Lösung eines Avidin-Meerrettich-Peroxidase-Konjugats (0,3 $\mu$g/ml)
11 mg 2,2-Azino-bis-(3-äthylbenzothiazolin-6-sulfonsäure)-Diammoniumsalz
20 ml Citrat/Phosphat-Puffer (0,05 M Citrat / 0,1 M $Na_2HPO_4$)
1 ml 30 % $H_2O_2$
100 ml PBS
200 ml PGT-20-Puffer (PBS enthaltend 0,2 % Gelatine und 0,05 % Tween 20).

**Patentansprüche**
**Patentansprüche für folgende Vertragsstaaten : DE, GB, FR, CH, LI, IT, NL, BE, SE, LU**

1.  Gereinigter Human-MIF, dadurch charakterisiert, dass er nur Proteine humanen Ursprungs, die Epitope aufweisen, die vom monoklonalen Antikörper 1C5 gegen Human-MIF erkannt und gebunden werden, enthält und in Standard-Testanordnungen, bei denen die Wanderung von Makrophagen gemessen wird, aktiv ist, und seine Einzelproteine.

2.  Gereinigter Human-MIF gemäss Anspruch 1, dadurch charakterisiert, dass er aus mindestens vier Einzelproteinen vom Molekulargewicht ca. 8, ca. 14, ca. 28 und ca. 45 kg/Mol, besteht.

3. Ein Protein gemäss Anspruch 1, dadurch charakterisiert, dass die N-terminale Aminosäure-Sequenz

$$X_1-Leu-Thr-Glu-Leu-Glu-Lys-Ala-Leu-$$
$$Asn-Ser-Ile-Ile-Asp-Val-Tyr-His-Lys-Tyr$$

lautet, worin die Bedeutung der Aminosäure $X_1$ nicht festgelegt ist.

4. Ein Protein gemäss Anspruch 1 oder 3, dadurch charakterisiert, dass die N-terminale Aminosäure-Sequenz

$$Met-Leu-Thr-Glu-Leu-Glu-$$
$$Lys-Ala-Leu-Asn-Ser-Ile-Ile-Asp-Val-Tyr-His-Lys-Tyr-Ser-Leu-Ile-$$
$$Lys-Gly-Asn-Phe-His-Ala-Val-Tyr-Arg-Asp-Asp-Leu-Lys-Lys-Leu-Leu-$$
$$Glu-Thr-Glu-X_{42}-Pro-Gln-Tyr-Ile-Arg-Lys-Lys-Gly-Ala-Asp-Val-Trp-$$
$$Phe-Lys-Glu-Leu-Asp-Ile-Asn-X_{62}-X_{63}-X_{64}-Ala-Val$$

lautet, worin die Bedeutung der Aminosäuren $X_{42}$, $X_{62}$, $X_{63}$ und $X_{64}$ nicht festgelegt ist, $X_{42}$ aber nur Ser oder Cys bedeuten kann.

5. Ein Protein gemäss Anspruch 1, 3 oder 4 mit einem ungefähren Molekulargewicht von 8 kg/Mol.

6. Ein Protein gemäss Anspruch 1 oder 3 mit einem ungefähren Molekulargewicht von 14 kg/Mol.

7. Verfahren zur Herstellung von gereinigtem Human-MIF und seiner Einzelproteine, dadurch gekennzeichnet, dass man eine Human-MIF enthaltende Lösung, gewünschtenfalls nach an sich bekannten Reinigungsschritten,
    a) mit einem Trägermaterial mit auf dem Human-MIF spezifischen monoklonalen Antikörper 1C5 in Kontakt bringt, ungebundene Proteine und andere Fremdstoffe entfernt, den an die Antikörper gebundenen Human-MIF selektiv abspaltet und isoliert und,
    b) wenn erwünscht, den gereinigten Human-MIF in seine Einzelproteine auftrennt.

8. Verfahren gemäss Anspruch 7, worin die Human-MIF enthaltende Lösung ein Zellkulturüberstand von mit Concanavalin A stimulierten und kultivierten mononukleären Zellen ist.

9. Verfahren gemäss Anspruch 7 oder 8, worin die Human-MIF enthaltende Lösung vorgängig einer Ultrafiltration unterworfen und aufkonzentriert wird.

10. Verfahren gemäss Anspruch 7, 8 oder 9, worin der in Schritt a) gereinigte Human-MIF durch präparative Natriumdodecylsulfat-Polyacrylamid-Gel-Elektrophorese in seine Einzelproteine getrennt wird.

11. Verfahren gemäss Anspruch 7, 8 oder 9, worin der in Schritt a) gereinigte Human-MIF durch präparative Gel-Filtration-HPLC (Hochdruck-Flüssigchromatographie) in seine Einzelproteine getrennt wird.

12. Monoklonale Antikörper gegen Human-Makrophagen-Migrationsinhibitionsfaktor (Human-MIF), sowie Derivate davon.

13. Monoklonale Antikörper und Derivate davon gemäss Anspruch 12, dadurch gekennzeichnet, dass sie von Maus/Maus-Hybridoma-Zellen produziert werden.

14. Monoklonale Antikörper und Derivate davon gemäss Anspruch 12, dadurch gekennzeichnet, dass sie von Ratte/Maus-Hybridoma-Zellen produziert werden.

EP 0 162 812 B1

**15.** Der monoklonale Antikörper 1C5 gemäss Anspruch 12.

**16.** Der monoklonale Antikörper 7D10 gemäss Anspruch 12.

**17.** Verfahren zur Herstellung von monoklonalen Antikörpern und Derivaten davon gemäss Anspruch 12, gekennzeichnet dadurch, dass man diese monoklonale Antikörper produzierende Hybridoma-Zellen
   a) in vitro kultiviert und aus den Kulturüberständen die monoklonalen Antikörper isoliert, oder
   b) in vivo in einem geeigneten Säugetier vermehrt und aus den Körperflüssigkeiten dieses Säugetiers die monoklonalen Antikörper isoliert, und,
   c) wenn erwünscht, einen erhaltenen monoklonalen Antikörper in ein Derivat davon überführt.

**18.** Verfahren gemäss Anspruch 17, dadurch gekennzeichnet, dass von Balb/c-Mäusen abstammende Hybridoma-Zellen in gegebenenfalls mit einem Kohlenwasserstoff vorbehandelte Balb/c-Mäuse injiziert, nach 8-10 Tagen diesen Tieren Aszites-Flüssigkeit entnommen und die Antikörper daraus durch Fällung mit Ammoniumsulfat und chromatographische Reinigung isoliert werden.

**19.** Hybridoma-Zellinien, dadurch gekennzeichnet, dass sie monoklonale Antikörper gegen Human-MIF produzieren.

**20.** Hybridoma-Zellinien gemäss Anspruch 19, dadurch gekennzeichnet, dass sie Hybride von Maus-Myeloma-Zellen und Maus-Lymphozyten sind.

**21.** Hybridoma-Zellinien gemäss Anspruch 19, dadurch gekennzeichnet, dass sie Hybride von Maus-Myeloma-Zellen und Ratte-Lymphozyten sind.

**22.** Die Hybridoma-Zellinie 1C5 gemäss Anspruch 19, die in der "Collection Nationale de Cultures de Microorganismes" des Institut Pasteur in Paris unter der Nummer I-316 hinterlegt ist.

**23.** Die Hybridoma-Zellinie 7D10 gemäss Anspruch 19, die in der "Collection Nationale de Cultures de Microorganismes" des Institut Pasteur in Paris unter der Nummer I-418 hinterlegt ist.

**24.** Verfahren zur Herstellung von Hybridoma-Zellen gemäss Anspruch 19, dadurch gekennzeichnet, dass man geeignete Säugetiere mit MIF oder MIF-Konjugaten immunisiert, dem Säugetier entnommene Antikörper-produzierende Zellen mit Myeloma-Zellen fusioniert, die erhaltenen Hybridoma-Zellen kloniert und diejenigen Zellklone, die die gewünschten monoklonalen Antikörper produzieren, selektioniert.

**25.** Verfahren gemäss Anspruch 24, dadurch gekennzeichnet, dass Antikörper-produzierende Zellen von Balb/c-Mäusen mit Myeloma-Zellen der Zellinien X63-Ag8.653 oder Sp2/0-Ag14 fusioniert werden.

**26.** Verfahren gemäss Anspruch 24, dadurch gekennzeichnet, dass Antikörper-produzierende Zellen von DA-Ratten mit Myeloma-Zellen der Zellinie X63-Ag8.653 fusioniert werden.

**27.** Verfahren gemäss Anspruch 24, dadurch gekennzeichnet, dass zur Immunisierung Konjugate von Human-MIF-haltigen Protein-Fraktionen mit einem immunogenen Träger eingesetzt werden.

**28.** Verfahren gemäss Anspruch 24, dadurch gekennzeichnet, dass zur Immunisierung Konjugate von Maus-MIF-haltigen Protein-Fraktionen mit einem immunogenen Träger eingesetzt werden.

**29.** Verwendung der monoklonalen Antikörper und Derivate gemäss Anspruch 12 zur qualitativen und quantitativen Bestimmung von Human-MIF.

**30.** Verwendung der monoklonalen Antikörper und Derivate in einem Radioimmunoassay gemäss Anspruch 29.

**31.** Verwendung der monoklonalen Antikörper und Derivate in einem Enzym-Immunoassay gemäss Anspruch 29.

20

**32.** Test-Kits zur qualitativen und quantitativen Bestimmung von Human-MIF, dadurch gekennzeichnet, dass sie monoklonale Antikörper und/oder Derivate gemäss Anspruch 12 und gegebenenfalls Hilfsmittel enthalten.

**33.** Test-Kits gemäss Anspruch 32 für einen Radioimmunoassay.

**34.** Test-Kits gemäss Anspruch 32 für einen Enzym-Immunoassay.

**35.** Verwendung der monoklonalen Antikörper und Derivate gemäss Anspruch 12 zur Reinigung von Human-MIF.

**36.** Pharmazeutische Präparate enthaltend eine therapeutisch wirksame Menge von gereinigtem Human-MIF oder seiner Einzelproteine gemäss Anspruch 1 und eine signifikante Menge eines pharmazeutischen Hilfsmittels.

**37.** Pharmazeutische Präparate enthaltend eine therapeutisch wirksame Menge monoklonaler Antikörper gegen Human-MIF oder deren Derivate gemäss Anspruch 12 und eine signifikante Menge eines pharmazeutischen Hilfsmittels.

**38.** Pharmazeutische Präparate gemäss Anspruch 36 in Form von Liposomen aus einem Gemisch von synthetischem Phosphatidylserin und Phosphatidylcholin in wässriger Dispersion.

**Patenansprüche für folgenden Vertragsstaat : AT**

**1.** Verfahren zur Herstellung von gereinigtem Human-Makrophagen-Migrationsinhibitionsfaktor (Human-MIF), der nur Proteine humanen Ursprungs enthält, die Epitope aufweisen, die vom monoklonalen Antikörper 1C5 gegen Human-MIF erkannt und gebunden werden, und in Standard-Testanordnungen, bei denen die Wanderung von Makrophagen gemessen wird, aktiv ist, und seiner Einzelproteine, dadurch gekennzeichnet, dass man eine Human-MIF enthaltende Lösung, gewünschtenfalls nach an sich bekannten Reinigungsschritten, mit einem Trägermaterial mit auf Human-MIF spezifischen monoklonalen Antikörpern in Kontakt bringt, ungebundene Proteine und andere Fremdstoffe entfernt, den an die Antikörper gebundenen Human-MIF selektiv abspaltet und isoliert und, wenn erwünscht, den gereinigten Human-MIF in seine Einzelproteine auftrennt.

**2.** Verfahren gemäss Anspruch 1, worin die Human-MIF enthaltende Lösung ein Zellkulturüberstand von mit Concanavalin A stimulierten und kultivierten mononukleären Zellen ist.

**3.** Verfahren gemäss Anspruch 1 oder 2, worin die Human-MIF enthaltende Lösung vorgängig einer Ultrafiltration unterworfen und aufkonzentriert wird.

**4.** Verfahren gemäss Anspruch 1, 2 oder 3, worin der gereinigte Human-MIF durch präparative Natriumdodecylsulfat-Polyacrylamid-Gel-Elektrophorese in seine Einzelproteine getrennt wird.

**5.** Verfahren gemäss Anspruch 1, 2 oder 3, worin der gereinigte Human-MIF durch präparative Gel-Filtration-HPLC (Hochdruck-Flüssigchromatographie) in seine Einzelproteine getrennt wird.

**6.** Verfahren gemäss einem der Ansprüche 1 bis 5 zur Herstellung eines Einzelproteins von Human-MIF mit der N-terminalen Aminosäure-Sequenz

$$X_1\text{-Leu-Thr-Glu-Leu-Glu-Lys-Ala-Leu-Asn-Ser-Ile-Ile-Asp-Val-Tyr-His-Lys-Tyr,}$$

worin die Bedeutung der Aminosäure $X_1$ nicht festgelegt ist.

**7.** Verfahren gemäss einem der Ansprüche 1 bis 5 zur Herstellung eines Einzelproteins vom Human-MIF mit der N-terminalen Aminosäure-Sequenz

$$\text{Met-Leu-Thr-Glu-Leu-Glu-Lys-Ala-Leu-Asn-Ser-Ile-Ile-Asp-Val-}$$
$$\text{Tyr-His-Lys-Tyr-Ser-Leu-Ile-Lys-Gly-Asn-Phe-His-Ala-Val-Tyr-Arg-Asp-}$$
$$\text{Asp-Leu-Lys-Lys-Leu-Leu-Glu-Thr-Glu-}X_{42}\text{-Pro-Gln-Tyr-Ile-Arg-Lys-Lys-}$$
$$\text{Gly-Ala-Asp-Val-Trp-Phe-Lys-Glu-Leu-Asp-Ile-Asn-}X_{62}\text{-}X_{63}\text{-}X_{64}\text{-Ala-Val,}$$

worin die Bedeutung der Aminosäuren $X_{42}$, $X_{62}$, $X_{63}$ und $X_{64}$ nicht festgelegt ist, $X_{42}$ aber nur Ser oder Cys bedeuten kann.

8. Verfahren gemäss einem der Ansprüche 1 bis 7 zur Herstellung eines Einzelproteins von Human-MIF mit einem ungefähren Molekulargewicht von 8 kg/Mol.

9. Verfahren gemäss einem der Ansprüche 1 bis 7 zur Herstellung eines Einzelproteins von Human-MIF mit einem ungefähren Molekulargewicht von 14 kg/Mol.

10. Verfahren zur Herstellung von monoklonalen Antikörpern gegen Human-Makrophagen-Migrationsinhibitionsfaktor (Human-MIF) und von Derivaten davon, gekennzeichnet dadurch, dass man diese monoklonale Antikörper produzierende Hybridoma-Zellen in vitro kultiviert und aus den Kulturüberständen die monoklonalen Antikörper isoliert oder in vivo in einem geeigneten Säugetier vermehrt und aus den Körperflüssigkeiten dieses Säugetiers die monoklonalen Antikörper isoliert, und wenn erwünscht, einen erhaltenen monoklonalen Antikörper in ein Derivat davon überführt.

11. Verfahren gemäss Anspruch 10, dadurch gekennzeichnet, dass von Balb/c-Mäusen abstammende Hybridoma-Zellen in gegebenenfalls mit einem Kohlenwasserstoff vorbehandelte Balb/c-Mäuse injiziert, nach 8-10 Tagen diesen Tieren Aszites-Flüssigkeit entnommen und die Antikörper daraus durch Fällung mit Ammoniumsulfat und chromatographische Reinigung isoliert werden.

12. Verfahren gemäss Anspruch 10 oder 11 zur Herstellung des monoklonalen Antikörpers 1C5.

13. Verfahren gemäss Anspruch 10 oder 11 zur Herstellung des monoklonalen Antikörpers 7D10.

14. Verfahren gemäss Anspruch 10 zur Herstellung eines Biotin-Konjugats des monoklonalen Antikörpers 7D10.

15. Hybridoma-Zellinien, dadurch gekennzeichnet, dass sie monoklonale Antikörper gegen Human-MIF produzieren.

16. Hybridoma-Zellinien gemäss Anspruch 15, dadurch gekennzeichnet, dass sie Hybride von Maus-Myeloma-Zellen und Maus-Lymphozyten sind.

17. Hybridoma-Zellinien gemäss Anspruch 15, dadurch gekennzeichnet, dass sie Hybride von Maus-Myeloma-Zellen und Ratte-Lymphozyten sind.

18. Die Hybridoma-Zellinie 1C5 gemäss Anspruch 15, die in der "Collection Nationale de Cultures de Microorganismes" des Institut Pasteur in Paris unter der Nummer I-316 hinterlegt ist.

19. Die Hybridoma-Zellinie 7D10 gemäss Anspruch 15, die in der "Collection Nationale de Cultures de Microorganismes" des Institut Pasteur in Paris unter der Nummer I-418 hinterlegt ist.

20. Verfahren zur Herstellung von Hybridoma-Zellen gemäss Anspruch 15, dadurch gekennzeichnet, dass man geeignete Säugetiere mit MIF oder MIF-Konjugaten immunisiert, dem Säugetier entnommene Antikörper-produzierende Zellen mit Myeloma-Zellen fusioniert, die erhaltenen Hybridoma-Zellen kloniert und diejenigen Zellklone, die die gewünschten monoklonalen Antikörper produzieren, selektioniert.

21. Verfahren gemäss Anspruch 20, dadurch gekennzeichnet, dass Antikörper-produzierende Zellen von Balb/c-Mäusen mit Myeloma-Zellen der Zellinien X63-Ag8.653 oder Sp2/0-Ag14 fusioniert werden.

22

**22.** Verfahren gemäss Anspruch 20, dadurch gekennzeichnet, dass Antikörper-produzierende Zellen von DA-Ratten mit Myeloma-Zellen der Zellinie X63-Ag8.653 fusioniert werden.

**23.** Verfahren gemäss Anspruch 20, dadurch gekennzeichnet, dass zur Immunisierung Konjugate von Human-MIF-haltigen Protein-Fraktionen mit einem immunogenen Träger eingesetzt werden.

**24.** Verfahren gemäss Anspruch 20, dadurch gekennzeichnet, dass zur Immunisierung Konjugate von Maus-MIF-haltigen Protein-Fraktionen mit einem immunogenen Träger eingesetzt werden.

**25.** Test-Kits zur qualitativen und quantitativen Bestimmung von Human-MIF, dadurch gekennzeichnet, dass sie monoklonale Antikörper und/oder Derivate hergestellt gemäss Anspruch 10 und gegebenenfalls Hilfsmittel enthalten.

**26.** Test-Kits gemäss Anspruch 25 für einen Radioimmunoassay.

**27.** Test-Kits gemäss Anspruch 25 für einen Enzym-Immunoassay.

**Claims**
**Claims for the following Contracting States : DE, GB, FR, CH, LI, IT, NL, BE, SE, LU**

**1.** Purified human MIF, characterised in that it comprises only proteins of human origin that have epitopes that are recognised and bound by the monoclonal antibody 1C5 to human MIF, and is active in standard test procedures in which the migration of macrophages is measured, and its individual proteins.

**2.** Purified human MIF according to claim 1, characterised in that it consists of at least four individual proteins of molecular weight approximately 8, approximately 14, approximately 28 and approximately 45 kg/mole.

**3.** A protein according to claim 1, characterised in that the N-terminal amino acid sequence is

$$X_1-Leu-Thr-Glu-Leu-$$
$$Glu-Lys-Ala-Leu-Asn-Ser-Ile-Ile-Asp-Val-Tyr-His-Lys-Tyr$$

in which the meaning of the amino acid $X_1$ is not fixed.

**4.** A protein according to claim 1 or 3, characterised in that the N-terminal amino acid sequence is

$$Met-Leu-Thr-$$
$$Glu-Leu-Glu-Lys-Ala-Leu-Asn-Ser-Ile-Ile-Asp-Val-Tyr-His-$$
$$Lys-Tyr-Ser-Leu-Ile-Lys-Gly-Asn-Phe-His-Ala-Val-Tyr-Arg-$$
$$Asp-Asp-Leu-Lys-Lys-Leu-Leu-Glu-Thr-Glu-X_{42}-Pro-Gln-Tyr-$$
$$Ile-Arg-Lys-Lys-Gly-Ala-Asp-Val-Trp-Phe-Lys-Glu-Leu-Asp-$$
$$Ile-Asn-X_{62}-X_{63}-X_{64}-Ala-Val$$

in which the meaning of the amino acids $X_{42}$, $X_{62}$, $X_{63}$ and $X_{64}$ is not fixed, but $X_{42}$ may represent only Ser or Cys.

**5.** A protein according to claim 1, 3 or 4 having an approximate molecular weight of 8 kg/mole.

**6.** A protein according to claim 1 or 3 having an approximate molecular weight of 14 kg/mole.

7. A process for the manufacture of purified human MIF and its individual proteins, characterised in that a solution comprising human MIF, if desired after purification steps that are known per se,
   a) is brought into contact with a carrier material having monoclonal antibodies 1C5 specific to human MIF, unbound proteins and other foreign substances are removed, the human MIF bound to the antibodies is selectively split off and isolated and,
   b) if desired, the purified human MIF is separated into its individual proteins.

8. A process according to claim 7 in which the solution comprising human MIF is a cell culture supernatant of mononuclear cells that have been stimulated and cultured with concanavalin A.

9. A process according to claim 7 or 8 in which the solution comprising human MIF is subjected beforehand to ultrafiltration and concentration.

10. A process according to claim 7, 8 or 9 in which the human MIF purified in step a) is separated into its individual proteins by preparative sodium dodecyl sulphate polyacrylamide gel electrophoresis.

11. A process according to claim 7, 8 or 9 in which the human MIF purified in step a) is separated into its individual proteins by preparative gel filtration-HPLC (high pressure liquid chromatography).

12. A monoclonal antibody to human macrophage migration inhibition factor (human MIF), or a derivative thereof.

13. A monoclonal antibody or a derivative thereof according to claim 12, characterised in that it is produced by mouse/mouse hybridoma cells.

14. A monoclonal antibody or a derivative thereof according to claim 12, characterised in that it is produced by rat/mouse hybridoma cells.

15. The monoclonal antibody 1C5 according to claim 12.

16. The monoclonal antibody 7D10 according to claim 12.

17. A process for the preparation of a monoclonal antibody or a derivative thereof according to claim 12, characterised in that hybridoma cells producing the monoclonal antibody
   a) are cultured in vitro and the monoclonal antibody is isolated from the culture supernatants, or
   b) are multiplied in vivo in a suitable mammal and the monoclonal antibody is isolated from the body fluids of that mammal, and,
   c) if desired, a resulting monoclonal antibody is converted into a derivative thereof.

18. A process according to claim 17, characterised in that hybridoma cells originating from Balb/c mice are injected into Balb/c mice that have optionally been pretreated with a hydrocarbon, after 8-10 days ascitic fluid is taken from these animals and the antibody is isolated therefrom by precipitation with ammonium sulphate and purification by chromatography.

19. A hybridoma cell line, characterised in that it produces a monoclonal antibody to human MIF.

20. A hybridoma cell line according to claim 19, characterised in that it is a hybrid of a mouse myeloma cell and a mouse lymphocyte.

21. A hybridoma cell line according to claim 19, characterised in that it is a hybrid of a mouse myeloma cell and a rat lymphocyte.

22. The hybridoma cell line 1C5 according to claim 19 which is deposited at the "Collection Nationale de Cultures de Microorganismes" of the Institut Pasteur in Paris under number I-316.

23. The hybridoma cell line 7D10 according to claim 19 which is deposited at the "Collection Nationale de Cultures de Microorganismes" of the Institut Pasteur in Paris under number I-418.

EP 0 162 812 B1

**24.** A process for the preparation of hybridoma cells according to claim 19, characterised in that a suitable mammal is immunised with MIF or an MIF conjugate, antibody-producing cells taken from the mammal are fused with myeloma cells, the resulting hybridoma cells are cloned and those cell clones which produce the desired monoclonal antibodies are selected.

**25.** A process according to claim 24, characterised in that antibody-producing cells of Balb/c mice are fused with myeloma cells of the cell lines X63-Ag8.653 or Sp2/0-Ag14.

**26.** A process according to claim 24, characterised in that antibody-producing cells of DA rats are fused with myeloma cells of the cell line X63-Ag8.653.

**27.** A process according to claim 24, characterized in that, for immunisation, a conjugate of a human MIF-containing protein fraction with an immunogenic carrier is used.

**28.** A process according to claim 24, characterised in that, for immunisation, a conjugate of a mouse MIF-containing protein fraction with an immunogenic carrier is used.

**29.** Use of a monoclonal antibody or a derivative according to claim 12 for the qualitative and quantitative determination of human MIF.

**30.** Use of a monoclonal antibody or a derivative in a radioimmunoassay according to claim 29.

**31.** Use of a monoclonal antibody or a derivative in an enzyme-immunoassay according to claim 29.

**32.** A test kit for the qualitative and quantitative determination of human MIF, characterised in that it comprises a monoclonal antibody and/or a derivative according to claim 12 and optionally adjuncts.

**33.** A test kit according to claim 32 for a radioimmunoassay.

**34.** A test kit according to claim 32 for an enzymeimmunoassay.

**35.** Use of a monoclonal antibody or a derivative according to claim 12 for the purification of human MIF.

**36.** A pharmaceutical preparation comprising a therapeutically effective amount of purified human MIF or its individual proteins according to claim 1 and a significant amount of a pharmaceutical adjunct.

**37.** A pharmaceutical preparation comprising a therapeutically effective amount of a monoclonal antibody to human MIF or a derivative thereof according to claim 12 and a significant amount of a pharmaceutical adjunct.

**38.** A pharmaceutical preparation according to claim 36 in the form of liposomes consisting of a mixture of synthetic phosphatidylserine and phosphatidylcholine in aqueous dispersion.

**Claims for the following Contracting State : AT**

**1.** A process for the manufacture of purified human macrophage migration inhibition factor (human MIF) comprising only proteins of human origin that have epitopes that are recognised and bound by the monoclonal antibody 1C5 to human MIF, and is active in standard test procedures in which the migration of macrophages is measured, and its individual proteins, characterised in that a solution comprising human MIF, if desired after purification steps that are known per se, is brought into contact with a carrier material having monoclonal antibodies specific to human MIF, unbound proteins and other foreign substances are removed, the human MIF bound to the antibodies is selectively split off and isolated and, if desired, the purified human MIF is separated into its individual proteins.

**2.** A process according to claim 1 in which the solution comprising human MIF is a cell culture supernatant of mononuclear cells that have been stimulated and cultured with concanavalin A.

**3.** A process according to claim 1 or 2 in which the solution comprising human MIF is subjected beforehand to ultrafiltration and concentration.

25

**3.** A process according to claim 1, 2 or 3 in which the purified human MIF is separated into its individual proteins by preparative sodium dodecyl sulphate polyacrylamide gel electrophoresis.

**5.** A process according to claim 1, 2 or 3 in which the purified human MIF is separated into its individual proteins by preparative gel filtration-HPLC (high pressure liquid chromatography).

**6.** A process according to any one of claims 1 to 5 for the preparation of an individual protein of human MIF having the N-terminal amino acid sequence

$$X_1\text{-Leu-Thr-}$$
$$\text{Glu-Leu-Glu-Lys-Ala-Leu-Asn-Ser-Ile-Ile-Asp-Val-Tyr-His-}$$
$$\text{Lys-Tyr}$$

in which the meaning of the amino acid $X_1$ is not fixed.

**7.** A process according to any one of claims 1 to 5 for the preparation of an individual protein of human MIF having the N-terminal amino acid sequence

$$\text{Met-Leu-Thr-}$$
$$\text{Glu-Leu-Glu-Lys-Ala-Leu-Asn-Ser-Ile-Ile-Asp-Val-Tyr-His-}$$
$$\text{Lys-Tyr-Ser-Leu-Ile-Lys-Gly-Asn-Phe-His-Ala-Val-Tyr-Arg-}$$
$$\text{Asp-Asp-Leu-Lys-Leu-Leu-Glu-Thr-Glu-}X_{42}\text{-Pro-Gln-Tyr-}$$
$$\text{Ile-Arg-Lys-Lys-Gly-Ala-Asp-Val-Trp-Phe-Lys-Glu-Leu-Asp-}$$
$$\text{Ile-Asn-}X_{62}\text{-}X_{63}\text{-}X_{64}\text{-Ala-Val}$$

in which the meaning of the amino acids $X_{42}$, $X_{62}$, $X_{63}$ and $X_{64}$ is not fixed, but $X_{42}$ may represent only Ser or Cys.

**8.** A process according to any one of claims 1 to 7 for the preparation of an individual protein of human MIF having an approximate molecular weight of 8 kg/mole.

**9.** A process according to any one of claims 1 to 7 for the preparation of an individual protein of human MIF having an approximate molecular weight of 14 kg/mole.

**10.** A process for the preparation of a monoclonal antibody to human macrophage migration inhibition factor (human MIF) or a derivative thereof, characterised in that hybridoma cells producing the monoclonal antibody are cultured in vitro and the monoclonal antibody is isolated from the culture supernatants, or are multiplied in vivo in a suitable mammal and the monoclonal antibody is isolated from the body fluids of that mammal, and, if desired, a resulting monoclonal antibody is converted into a derivative thereof.

**11.** A process according to claim 10, characterised in that hybridoma cells originating from Balb/c mice are injected into Balb/c mice that have optionally been pretreated with a hydrocarbon, after 8-10 days ascitic fluid is taken from these animals and the antibody is isolated therefrom by precipitation with ammonium sulphate and purification by chromatography.

**12.** A process according to claim 10 or 11 for the manufacture of the monoclonal antibody 1C5.

**13.** A process according to claim 10 or 11 for the manufacture of the monoclonal antibody 7D10.

**14.** A process according to claim 10 for the manufacture of a biotin conjugate of the monoclonal antibody 7D10.

**15.** A hybridoma cell line, characterised in that it produces a monoclonal antibody to human MIF.

**16.** A hybridoma cell line according to claim 15, characterised in that it is a hybrid of a mouse myeloma cell and a mouse lymphocyte.

**17.** A hybridoma cell line according to claim 15, characterised in that it is a hybrid of a mouse myeloma cell and a rat lymphocyte.

**18.** The hybridoma cell line 1C5 according to claim 15 which is deposited at the "Collection Nationale de Cultures de Microorganismes" of the Institut Pasteur in Paris under number I-316.

**19.** The hybridoma cell line 7D10 according to claim 15 which is deposited at the "Collection Nationale de Cultures de Microorganismes" of the Institut Pasteur in Paris under number I-418.

**20.** A process for the preparation of hybridoma cells according to claim 15, characterised in that a suitable mammal is immunised with MIF or a MIF conjugate, antibody-producing cells taken from the mammal are fused with myeloma cells, the resulting hybridoma cells are cloned and those cell clones which produce the desired monoclonal antibodies are selected.

**21.** A process according to claim 20, characterised in that antibody-producing cells of Balb/c mice are fused with myeloma cells of the cell lines X63-Ag8.653 or Sp2/0-Ag14.

**22.** A process according to claim 20, characterised in that antibody-producing cells of DA rats are fused with myeloma cells of the cell line X63-Ag8.653.

**23.** A process according to claim 20, characterised in that, for immunisation, a conjugate of a human MIF-containing protein fraction with an immunogenic carrier is used.

**24.** A process according to claim 20, characterised in that, for immunisation, a conjugate of a mouse MIF-containing protein fraction with an immunogenic carrier is used.

**25.** A test kit for the qualitative and quantitative determination of human MIF, characterised in that it comprises a monoclonal antibody and/or a derivative prepared according to claim 10 and optionally adjuncts.

**26.** A test kit according to claim 25 for a radioimmunoassay.

**27.** A test kit according to claim 25 for an enzymeimmunoassay.

**Revendications**

**Revendications pour les Etats contractants suivants : DE, GB, FR, CH, LI, IT, NL, BE, SE, LU**

**1.** Facteur d'inhibition de la migration des macrophages humains (Human-MIF) purifié, caractérisé en ce qu'il ne contient que des protéines d'origine humaine, qui présentent des épitopes qui sont reconnus et sont liés par l'anticorps monoclonal 1C5 anti-Human-MIF, et qui est actif dans des dispositifs expérimentaux standards dans lesquels on mesure la migration des macrophages, et ses protéinez isolées.

**2.** Human-MIF purifié selon la revendication 1, caractérisé en ce qu'il se compose d'au moins quatre protéines isolées d'un poids moléculaire d'environ 8, d'environ 14, d'environ 28 et d'environ 45 kg/mol.

**3.** Protéine selon la revendication 1, caractérisé en ce que la séquence d'acides aminés N-terminale est

$$X_1-Leu-Thr-Glu-Leu-Glu-Lys-Ala-Leu-$$
$$Asn-Ser-Ile-Ile-Asp-Val-Tyr-His-Lys-Tyr$$

où la détermination de l'acide aminé $X_1$ n'est pas établie.

**4.** Protéine selon la revendication 1 ou 3, caractérisée en ce que la séquence d'acides aminés N-terminale est

$$Met-Leu-Thr-Glu-Leu-Glu-$$
$$Lys-Ala-Leu-Asn-Ser-Ile-Ile-Asp-Val-Tyr-His-Lys-Tyr-Ser-Leu-Ile-$$
$$Lys-Gly-Asn-Phe-His-Ala-Val-Tyr-Arg-Asp-Asp-Leu-Lys-Lys-Leu-Leu-$$
$$Glu-Thr-Glu-X_{42}-Pro-Gln-Tyr-Ile-Arg-Lys-Lys-Gly-Ala-Asp-Val-Trp-$$
$$Phe-Lys-Glu-Leu-Asp-Ile-Asn-X_{62}-X_{63}-X_{64}-Ala-Val$$

où la signification des acides aminés $X_{42}$, $X_{62}$, $X_{63}$ et $X_{64}$ n'est pas déterminée, mais $X_{42}$ ne peut être que Ser ou Cys.

**5.** Protéine selon la revendication 1, 3 ou 4, ayant un poids moléculaire approché de 8 kg/mol.

EP 0 162 812 B1

**6.** Protéine selon la revendication 1 ou 3, ayant un poids moléculaire approché de 14 kg/mol.

**7.** Procédé de préparation de Human-MIF purifié et de ses protéines isolées, caractérisé en ce qu'on traite une solution contenant du Human-MIF, si on le désire après des étapes de purification connues, de la façon suivante,

a) on la met en contact avec un support avec l'anticorps monoclonal 1C5 spécifique du Human-MIF, on retire les protéines non-liées et les autres corps étrangers, on sépare sélectivement et on isole le Human-MIF lié aux anticorps, et

b) si on le désire, on sépare le Human-MIF purifié en ses protéines isolées.

**8.** Procédé selon la revendication 7, dans lequel la solution contenant le Human-MIF est un surnageant de culture cellulaire de cellules mononucléaires stimulées à la concanavaline A et cultivées.

**9.** Procédé selon la revendication 7 ou 8, dans lequel on soumet au préalable la solution contenant le Human-MIF à une ultrafiltration et on concentre.

**10.** Procédé selon la revendication 7, 8 ou 9, où le Human-MIF purifié dans l'étape a) est séparé en ses protéines isolées par électrophorèse préparative sur gel de dodécylsulfate de sodium-polyacrylamide.

**11.** Procédé selon la revendication 7, 8 ou 9, dans lequel le Human-MIF purifié dans l'étape a) est séparé de ses protéinez isolées par HPLC préparative avec filtration sur gel (chromatographie en phase liquide haute pression).

**12.** Anticorps monoclonaux contre le facteur d'inhibition de la migration des macrophages humains (Human-MIF), ainsi que leurs dérivés.

**13.** Anticorps monoclonaux et leurs dérivés selon la revendication 12, caractérisés en ce qu'ils sont produits par des cellules d'hybridome souris/souris.

**14.** Anticorps monoclonaux et leurs dérivés selon la revendication 12, caractérisés en ce qu'ils sont produits par des cellules d'hybridome de rat/souris.

**15.** L'anticorps monoclonal 1C5 selon la revendication 12.

**16.** L'anticorps monoclonal 7D10 selon la revendication 12.

**17.** Procédé de préparation d'anticorps monoclonaux ou de leurs dérivés selon la revendication 12, caractérisé en ce qu'on soumet ces cellules d'hybridomes productrices d'anticorps monoclonaux au traitement suivant :

a) on les cultive in vitro et on isole les anticorps monoclonaux à partir des surnageants de culture, ou

b) on les multiplie in vivo chez un mammifère approprié et on isole les anticorps monoclonaux à partir des liquides corporels de ce mammifère et

c) si on le désire, on transforme un anticorps monoclonal obtenu en un de ses dérivés.

**18.** Procédé selon la revendication 17, caractérisé en ce qu'on injecte des cellules d'hybridome provenant de souris Balb/c à des souris Balb/c éventuellement prétraitées avec un hydrocarbure, en ce qu'au bout de 8 à 10 jours on prélève le liquide d'ascite de ces animaux et on en isole les anticorps par précipitation avec du sulfate d'ammonium et purification chromatographique.

**19.** Lignées cellulaires d'hybridome, caractérisées en ce qu'elles produisent des anticorps monoclonaux anti-Human-MIF.

**20.** Lignées cellulaires d'hybridome selon la revendication 19, caractérisées en ce qu'elles sont des hybrides de myélome de souris et de lymphocytes de souris.

**21.** Lignées cellulaires d'hybridome selon la revendication 19, caractérisées en ce qu'elles sont des hybrides de myélome de souris et de lymphocytes de rat.

28

**22.** Lignée cellulaire d'hybridome selon la revendication 19, qui a été déposée à la Collection Nationale de Culture de Microorganismes de l'Institut Pasteur à Paris sous le numéro I-316.

**23.** Lignée cellulaire d'hybridome 7D10 selon la revendication 19, qui a été déposée à la Collection Nationale de Culture de Microorganismes de l'Institut Pasteur à Paris sous le numéro I-418.

**24.** Procédé de préparation de cellules d'hybridome selon la revendication 19, caractérisé en ce qu'on immunise des mammifères appropriés avec du MIF ou du conjugué de MIF, en ce qu'on fusionne les cellules productrices d'anticorps prélevées chez les mammifères avec des cellules de myélome, en ce qu'on clone les cellules d'hybridome obtenues et en ce qu'on sélectionne les cellules qui produisent les anticorps monoclonaux désirés.

**25.** Procédé selon la revendication 24, caractérisé en ce qu'on fusionne les cellules productrices d'anticorps de souris Balb/c avec des cellules de myélome des lignées cellulaires X-3-Ag8.653 ou Sp2/0-Ag14.

**26.** Procédé selon la revendication 24, caractérisé en ce qu'on fusionne des cellules productrices d'anticorps de rat DA avec des cellules de myélome de lignée cellulaire X63-Ag8.653.

**27.** Procédé selon la revendication 24, caractérisé en ce qu'on utilise pour l'immunisation des conjugués de fraction protéique contenant du Human-MIF avec un support immunogène.

**28.** Procédé selon la revendication 24, caractérisé en ce qu'on utilise pour l'immunisation du conjugué de fractions protéiques contenant du MIF de souris avec un support immunogène.

**29.** Utilisation des anticorps monoclonaux et dérivés selon la revendication 12 pour la détermination qualitative et quantitative du Human-MIF.

**30.** Application des anticorps monoclonaux et de leurs dérivés dans un radioimmunodosage selon la revendication 29.

**31.** Application des anticorps monoclonaux et de leurs dérivés dans un dosage immunoenzymatique selon la revendication 29.

**32.** Nécessaires expérimentaux pour la détermination qualitative et quantitative de Human-MIF, caractérisés en ce qu'ils contiennent de anticorps monoclonaux et/ou leurs dérivés selon la revendication 12 et éventuellement des additifs.

**33.** Nécessaires expérimentaux selon la revendication 32 pour radioimmunodosage.

**34.** Nécessaires expérimentaux selon la revendication 32 pour immunodosage enzymatique.

**35.** Application des anticorps monoclonaux et de leurs dérivés selon la revendication 12 à la purification du Human-MIF.

**36.** Préparation pharmaceutique contenant une quantité thérapeutiquement active de Human-MIF purifié ou ses protéines isolées selon la revendication 1, et une quantité significative d'un adjuvant pharmaceutique.

**37.** Préparations pharmaceutiques contenant une quantité thérapeutiquement active d'anticorps monoclonaux anti-Human-MIF ou de leurs dérivés selon la revendication 12 et une quantité significative d'un adjuvant pharmaceutique.

**38.** Préparations pharmaceutiques selon la revendication 36 sous forme de liposomes faits d'un mélange de phosphatidylsérine et de phosphatidylcholine de synthèse en dispersion aqueuse.

EP 0 162 812 B1

**Revendications pour l'Etat contactant suivant : AT**

1. Procédé de préparation de facteur d'inhibition de la migration des macrophages humains (Human-MIF) purifié qui ne contient que des protéines d'origine humaine, qui présente des épitopes qui sont reconnus et liés par l'anticorps monoclonal 1C5 anti-Human-MIF, et qui est actif dans des dispositifs expérimentaux standards dans lesquels on mesure la migration des macrophages, et ses protéines isolées, caractérisé en ce qu'on met une solution contenant le Human-MIF, éventuellement après les étapes de purification connues, en contact avec un support avec des anticorps monoclonaux spécifiques du Human-MIF, en ce qu'on retire les protéines non-liées et autres corps étrangers, en ce qu'on clive et qu'on isole sélectivement le Human-MIF lié aux anticorps et, si on le désire, en ce qu'on sépare le Human-MIF purifié en ses protéines isolées.

2. Procédé selon la revendication 1, dans lequel la solution contenant le Human-MIF est un surnageant de culture cellulaire de cellules mononucléaires stimulées avec la concanavaline A et cultivées.

3. Procédé selon la revendication 1 ou 2, dans lequel on soumet au préalable la solution contenant le Human-MIF à une ultrafiltration et on la concentre.

4. Procédé selon la revendication 1, 2 ou 3, dans lequel on sépare le Human-MIF purifié en se protéines isolées, par électrophorèse préparative sur gel de dodécylsulfate de sodium-polyacrylamide.

5. Procédé selon la revendication 1, 2 ou 3, dans lequel on sépare en ses protéines isolées le Human-MIF purifié par HPLC préparative avec filtration sur gel (chromatographie en phase liquide haute pression).

6. Procédé selon l'une quelconque des revendications 1 à 5, de préparation d'une protéine isolée de Human-MIF avec la séquence d'acides aminés N-terminale

$$X_1-Leu-Thr-Glu-\overset{5}{Leu}-Glu-Lys-Ala-Leu-\overset{10}{Asn}-Ser-Ile-Ile-Asp-\overset{15}{Val}-$$
$$Tyr-His-Lys-Tyr,$$

où la signification de l'acide aminé $X_1$ n'est pas établie.

7. Procédé selon l'une des revendications 1 à 5 de préparation de protéines isolées de Human-MIF à séquences d'acides aminés N-terminales :

$$Met-Leu-Thr-Glu-\overset{5}{Leu}-Glu-Lys-Ala-Leu-\overset{10}{Asn}-Ser-Ile-Ile-Asp-\overset{15}{Val}-$$
$$Tyr-His-Lys-Tyr-\overset{20}{Ser}-Leu-Ile-Lys-Gly-\overset{25}{Asn}-Phe-His-Ala-Val-\overset{30}{Tyr}-Arg-Asp-$$
$$Asp-\overset{35}{Leu}-Lys-Lys-Leu-Leu-\overset{40}{Glu}-Thr-Glu-X_{42}-Pro-\overset{45}{Gln}-Tyr-Ile-Arg-Lys-Lys-$$
$$\overset{50}{Gly}-Ala-Asp-Val-Trp-\overset{55}{Phe}-Lys-Glu-Leu-Asp-\overset{60}{Ile}-Asn-X_{62}-X_{63}-X_{64}-Ala-Val,$$

où la signification des acides aminés $X_{42}$, $X_{62}$, $X_{63}$ et $X_{64}$ n'est pas établie, mais $X_{42}$ ne peut représenter que Ser ou Cys.

8. Procédé selon l'une des revendications 1 à 7 de préparation d'une protéine isolée de Human-MIF ayant un poids moléculaire approximatif de 8 kg/mol.

9. Procédé selon l'une des revendications 1 à 7 de préparation d'une protéine isolée de Human-MIF ayant un poids moléculaire approximatif de 14 kg/mol.

10. Procédé de préparation d'anticorps monoclonaux contre le facteur d'inhibition de la migration des macrophages humains (Human-MIF) et de leurs dérivés, caractérisé en ce qu'on cultive in vitro des

30

cellules d'hybridomes productrices de ces anticorps monoclonaux et en ce qu'à partir des surnageants de culture on isole les anticorps monoclonaux ou on les multiplie in vivo chez un mammifère approprié et en ce qu'à partir des liquides corporels de ce mammifère on isole les anticorps monoclonaux et, si on le désire, en ce qu'on transforme un anticorps monoclonal obtenu en un de ses dérivés.

11. Procédé selon la revendication 10, caractérisé en ce qu'on injecte des cellules d'hybridome provenant de souris Balb/c à des souris Balb/c éventuellement préalablement traitées avec un hydrate de carbone, en ce qu'au bout de 8 à 10 on prélève le liquide d'ascite de ces animaux et en ce qu'on en isole les anticorps par précipitation avec du sulfate d'ammonium et purification chromatographique.

12. Procédé selon la revendication 10 ou 11 de préparation de l'anticorps monoclonal 1C5.

13. Procédé selon la revendication 10 ou 11 de préparation de l'anticorps monoclonal 7D10.

14. Procédé selon la revendication 10 de préparation d'un conjugué de biotine de l'anticorps monoclonal 7D10.

15. Lignées cellulaires d'hybridome caractérisées en ce qu'elle produisent des anticorps monoclonaux anti-Human-MIF.

16. Lignées cellulaires selon la revendication 15, caractérisées en ce qu'elles sont des hybrides de cellules de myélome de souris et de lymphocyte de souris.

17. Lignées cellulaires d'hybridome selon la revendication 15, caractérisé en ce que ce sont des hybrides de cellules de myélome de souris et de lymphocytes de rat.

18. Lignée cellulaire d'hybridome 1C5 selon la revendication 15, qui est déposée à la Collection Nationale de Culture de Microorganismes de l'Institut Pasteur de PARIS sous le numéro I-316.

19. Signée cellulaire d'hybridome 7D10 selon la revendication 15, qui est déposée à la Collection Nationale de Culture de Microorganismes de l'Institut Pasteur de Paris sous le numéro I-418.

20. Procédé de préparation de cellules d'hybridome selon la revendication 15, caractérisé en ce qu'on immunise des mammifères appropriés avec du MIF ou des conjugués de MIF, en ce qu'on fusionne des cellules productrices d'anticorps prélevées chez les mammifères avec des cellules de myélome, en ce qu'on clone les cellules d'hybridome obtenues et en ce qu'on sélectionne les clones cellulaires qui produisent les anticorps monoclonaux désirés.

21. Procédé selon la revendication 20, caractérisé en ce qu'on fusionne des cellules productrices d'anticorps de souris Balb/c avec des cellules de myélome des lignées cellulaires X63-Ag8.653 ou Sp2/O-Ag14.

22. Procédé selon la revendication 20, caractérisé en ce qu'on fusionne des cellules productrices d'anticorps de rats DA avec des cellules de myélome de lignée cellulaire X63-Ag8.653.

23. Procédé selon la revendication 20, caractérisé en ce qu'on utilise pour l'immunisation des conjugués de fractions protéiques contenant le Human-MIF avec un support immunogène.

24. Procédé selon la revendication 20, caractérisé en ce qu'on utilise pour l'immunisation des conjugués de fractions protéiques contenant le MIF de souris avec un support immunogène.

25. Nécessaires expérimentaux pour la détermination qualitative et quantitative de Human-MIF, caractérisés en ce qu'ils contiennent des anticorps monoclonaux et/ou leurs dérivés préparés selon la revendication 10 et éventuellement des adjuvants.

26. Nécessaires expérimentaux selon la revendication 25 pour radioiimmunodosage.

27. Nécessaires expérimentaux selon la revendication 25, pour immunodosage enzymatique.